(19) Europäisches Patentamt
European Patent Office
Office européen des brevets

(11) Numéro de publication: **0 010 029**
**B1**

(12) **FASCICULE DE BREVET EUROPEEN**

(45) Date de publication du fascicule du brevet: **17.11.82**

(21) Numéro de dépôt: **79400666.8**

(22) Date de dépôt: **19.09.79**

(51) Int. Cl.³: **A 61 K 31/475, C 07 D 471/04 //C07D209/88, (C07D471/04, 221/00, 209/00)**

(54) **Ellipticines substituées en position 1 par une chaîne polyaminée, leur synthèse et médicaments les contenant.**

(30) Priorité: **21.09.78 FR 7827137**

(43) Date de publication de la demande: **16.04.80 Bulletin 80/8**

(45) Mention de la délivrance du brevet: **17.11.82 Bulletin 82/46**

(84) Etats contractants désignés: **BE CH DE GB IT LU NL**

(56) Documents cités:
**FR - A - 1 556 174**

**COMPTES RENDUS DES SEANCES DE L'ACADEMIE DES SCIENCES, tôme 284, série C, 1977, Paris (FR) D. ROUSSELLE et al. "Nouvelle voie d'accès aux dérivés de l'ellipticine et analogues", pages 377—380.**

**SYNTHESIS, no. 7, juillet 1977, Stuttgart (DE) M. SAINSBURY "The synthesis of 6H-pyrido(4,3-b)carbazoles", pages 437—448.**

**JOURNAL OF MEDICINAL CHEMISTRY, vol. 18, no. 7, 1975, Columbus, Ohio, USA R.W. GUTHRIE et al. "Ellipticine derivatives", pages 755—760.**

(73) Titulaire: **ANVAR Agence Nationale de Valorisation de la Recherche
13, rue Madeleine Michelis
F-92522 Neuilly-sur-Seine (FR)**

(72) Inventeur: **Bisagni, Emile
16, rue Bossuet
F-91400 Orsay (FR)**
Inventeur: **Ducrocq, Claire
14, allée des Bathes
F-91400 Orsay-Les Ulis (FR)**
Inventeur: **Rivalle, Christian
78, rue des Casseaux
F-91120 Villebon/Yvette (FR)**
Inventeur: **Tambourin, Pierre
14, allée des Bathes
F-91400 Orsay-Les Ulis (FR)**
Inventeur: **Wendling, Françoise
24, rue Monge
F-75005 Paris (FR)**
Inventeur: **Civier, Alain
Chemin des Bourguignons Longpont/Orge
F-91310 Montlhérie (FR)**
Inventeur: **Montagnier, Luc
21, rue de Malabry
F-92350 Le Plessis-Robinson (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 010 029 B1

(72) Inventeur: **Chermann, Jean-Claude**
**2, Clos d'Ergal**
**F-78310 Elancourt (FR)**
Inventeur: **Gruest, Jacqueline**
**6, rue du Gué**
**F-94240 l'Hay-les-Roses (FR)**
Inventeur: **Lidereau, Rosette**
**59, rue Jules Michelet**
**F-92700 Colombes (FR)**

(74) Mandataire: **Harlé, Robert et al,**
**c/o Cabinet Harlé & Phélip 21, rue de la Rochefoucauld**
**F-75009 Paris (FR)**

# Ellipticines substituées en position 1 par une chaîne polyaminée, leur synthèse et médicaments les contenant

L'invention concerne la synthèse d'une nouvelle famille de produits, qui sont des pyrido[4,3-b]carbazoles (ellipticines) diversement substitués sur leur sommet 1. L'invention a aussi pour objet un procédé d'obtention de tels produits à partir de pyrido [4,3-b]carbazoles eux-mêmes préparés par une voie d'accès originale. En outre, l'invention est relative à l'application à titre de médicaments de ces nouveaux produits, en particulier pour le traitement des leucémies et des tumeurs. C'est ainsi que l'invention se rapporte aussi à des compositions pharmaceutiques contenant, à titre d'agents actifs, au moins l'un desdits produits.

De nombreux travaux ont été consacrés, au cours de ces dernières années, à la famille de l'ellipticine et de ses dérivés, dont on avait constaté l'activité antitumorale. Divers procédés ont été décrits dans l'art antérieur pour préparer des pyrido[4,3-b]carbazoles (ellipticines). A titre de référence bibliographique, on peut par exemple citer l'article de M. SAINSBURY, dans Synthesis, 1977, p. 437 et suivantes. Il importe toujours, cependant, de trouver de nouveaux composés doués d'une activité supérieure ou de mettre au point de nouvelles voies de synthèse.

La présente invention concerne ainsi de nouveaux pyrido[4,3-b]carbazoles (ellipticines) substitués en position 1 par une chaîne polyaminée et répondant à la formule générale (I):

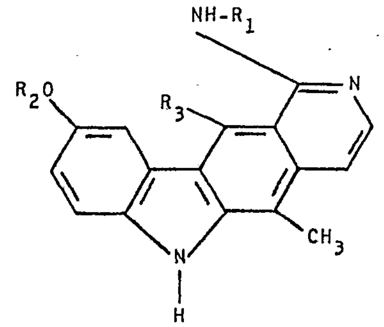

(I)

dans laquelle $R_1$ est un groupement $Y—(CH_2)_n—NR_4R_5$ où Y représente une liaison simple ou le groupe:

$$—\underset{\displaystyle CH_3}{\underset{|}{CH}}—$$

$R_4$ et $R_5$, identiques ou différents, sont l'hydrogène ou un radical alkyle inférieur, ou encore forment ensemble un cycle pouvant comporter 1 ou 2 atomes d'azote comme hétéroatomes et *n* est un nombre allant de 1 à 10, notamment de 2 à 7, $R_2$ est un atome d'hydrogène, un groupe alkyle inférieur ou un groupe benzyle et $R_3$ est un atome d'hydrogène ou un groupe $—CH_3$.

Au sens de la présente description, on désigne par "alkyle inférieur" tout groupement hydrocarboné de formule $C_xH_{2x+1}$ dans lequel *x* est un nombre entier allant de 1 à 4, par exemple les radicaux méthyle, éthyle, butyle, propyle.

L'invention concerne également les sels pharmaceutiquement acceptables des dérivés définis ci-dessus, obtenus avec des acides minéraux ou organiques couramment utilisés pour la formation de sels pharmaceutiquement acceptables.

L'invention concerne également un procédé pour l'obtention des nouveaux composés de formule (I) en passant par des chloro-1 pyrido[4,3-b]carbazoles.

Le procédé de l'invention implique une succession d'étapes qui sont illustrées par le schéma réactionnel représenté au dessin annexé. Dans ce qui suit, on décrira chacune des étapes.

*Première étape*

Dans cette étape, on fait réagir un chlorure de phényl (substitué en 4) diazonium de formule:

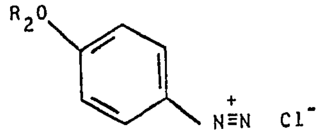

où $R_2$ a la signification indiquée précédemment, sur un cyclohexène à substitution amine en position 1, par exemple un N-morpholino-1, cyclohexène de formule:

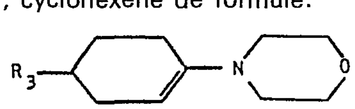

où $R_3$ a la signification indiquée (H ou $—CH_3$).

En vue de la réaction, il est avantageux de préparer in situ le sel de diazonium en faisant réagir en milieu acide un nitrite, tel que le nitrite de sodium, sur une phénylamine substituée en position 4 par le groupe $—OR_2$. Cette réaction se fait à température inférieure à la température ambiante, par exemple aux environs de 0°C. On peut alors faire réagir dans le même milieu réactionnel le cyclohexène à substitution amine en position 1. On utilise un N-morpholino-1 cyclohexène, un N-pipéridino-1 cyclohexène ou autre cyclohexène substitué en 1 par un cycle aminé. On obtient ainsi une monoarylhydrazone de cyclohexane dione-1,2 (formule 1 sur le schéma réactionnel annexé).

*Deuxième étape.*

Dans cette étape, on transforme la monoarylhydrazone obtenue à la première étape, de formule:

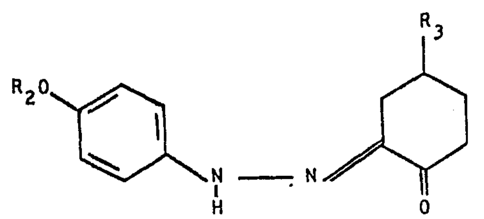

I

où $R_2$ et $R_3$ ont la signification indiquée, par indolisation à chaud et en milieu acide fort. La température de réaction dépend du milieu choisi et se situe en général entre 80°C et 200°C environ. Si l'on travaille en milieu solvant, on peut opérer à la température de reflux du solvant, par exemple, dans le cas de l'éthanol, aux environs de 80°C, ou dans le cas de l'eau, vers 100°C.

On obtient ainsi un oxo-1 tétrahydro-1,2,3,4 carbazole (formule 2 sur le schéma réactionnel annexé).

*Troisième étape.*

Dans cette étape, on acyle l'oxo-1 tétrahydro-1,2,3,4 carbazole obentu à la deuxième étape, de formule:

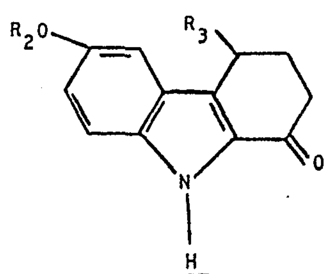

2

où $R_2$ et $R_3$ ont la signification indiquée, à l'aide de formiate d'éthyle en présence d'une base forte, ce qui conduit à un oxo-1 hydroxyméthylène-2-térahydro-1,2,3,4 carbazole (formule 3 sur le schéma réactionnel annexé).

A titre de base forte, on peut utiliser l'hydrure de sodium, l'éthoxyde de sodium ou une base forte analogue utilisée de façon classique dans les réactions de chimie organique. La température de réaction dépendra de la base utilisée. Si l'on augmente la température, la vitesse de réaction croît.

*Quatrième étape.*

Dans cette étape, on éthérifie l'oxo-1 hydroxyméthylène-2 tétrahydro-1,2,3,4 carbazole, de formule:

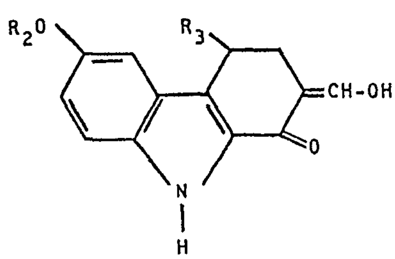

3

où $R_2$ et $R_3$ ont la signification indiquée, à l'aide d'un halogénure d'alkyle approprié, de préférence d'iodure d'isopropyle. La réaction se fait bien en milieu basique (carbonate de potassium, par exemple) dans un solvant aprotique (diméthyl-formamide, par exemple). On peut commencer la réaction à froid, par exemple vers 0—5°C, puis terminer à température ambiante.

On obtient ainsi un oxo-1 alkyloxyméthylène-2 dihydro-3,4 carbazole, en particulier l'oxo-1 isopropoxyméthylène-2 dihydro-3,4 carbazole (formule 4 sur le schéma réactionnel annexé).

*Cinquième étape.*

Dans cette étape, on traite l'oxo-1 isopropoxyméthylène-2 dihydro-3,4 carbazole, obtenu à la quatrième étape, de formule:

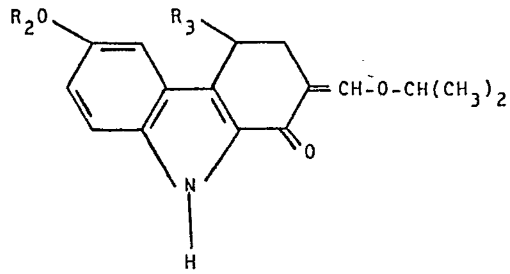

4

où $R_2$ et $R_3$ ont la signification indiquée, par quatre équivalents molaires de méthyl-lithium, après quoi on opère successivement une hydrolyse acide et une hydrolyse alcaline, celle-ci étant réalisée à température ambiante. La réaction avec le méthyl-lithium et l'hydrolyse acide se font le mieux à froid, par exemple vers 0°C. On obtient ainsi un méthyl-1 formyl-2 dihydro 3,4 carbazole (formule 5 sur le schéma réactionnel annexé).

*Sixième étape.*

Dans cette étape, on traite le méthyl-1 formyl-2 dihydro 3,4 carbazole obtenue à la cinquième étape, de formule:

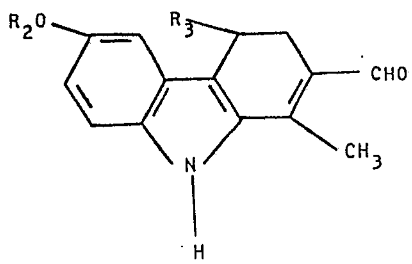

5

où $R_2$ et $R_3$ ont la signification indiquée, par du charbon palladié ou mieux par un dioxyde de manganèse, afin d'obtenir l'aromatisation du composé de départ, ce qui conduit à l'aldéhyde de formule 6 (voir schéma réactionnel annexé).

La réaction se fait en milieu solvant, par exemple un solvant benzénique, à une température de 70°C à 120°C environ. Dans le cas du benzène, on peut opérer á la température de reflux, soit environ 80°C.

*Septième étape.*

Dans cette étape, on traite l'aldehyde, obtenu à la sixième étape, de formule:

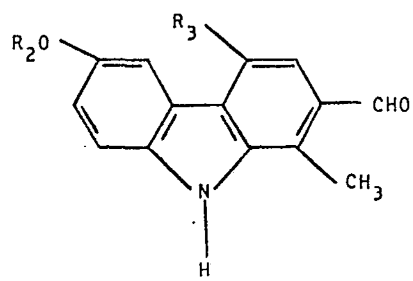

6

par l'acide malonique pour conduire à un acide acrylique (formule 7 sur le schéma réactionnel). Il convient d'exécuter la réaction en présence d'une quantité catalytique de pipéridine et à chaud. On travaille avantageusement dans la pyridine à l'ébullition.

*Huitième étape.*

Dans cette étape, on traite l'acide acrylique obtenu à la septième étape, de formule:

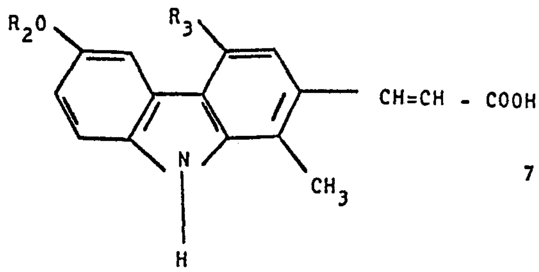

7

par le chloroformiate d'éthyle puis par l'azoture de sodium, selon la technique connue dite des anhydrides mixtes, ce qui conduit à l'azide correspondant (formule 8 sur le schéma réactionnel). Cette réaction se fait à froid, en milieu solvant tel que l'acétone.

*Neuvième étape.*

Dans cette étape, on traite l'azide obtenu à la huitième étape, de formule:

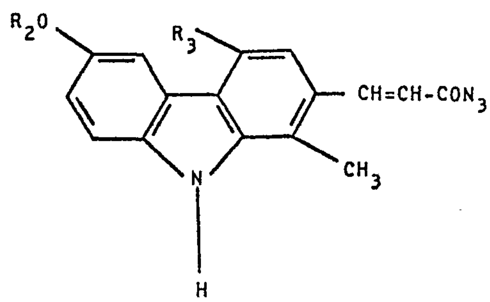

8

par le diphényl-éther à chaud, de préférence à la température d'ébullition, ce qui conduit à un dihydro-1,2 oxo-1 méthyl-5 pyrido[4,3-b]carbazole (formule 9 sur le schéma réactionnel).

*Dixième étape.*

Dans cette étape, on traite le dihydro-1,2 oxo-1 méthyl-5 pyrido[4,3-b]carbazole, obtenu à la neuvième étape, de formule:

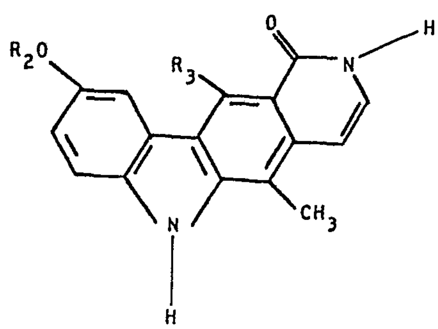

9

par l'oxychlorure de phosphore à chaud, et de préférence à la température d'ébullition, ce qui conduit à un chloro-1 méthyl-5 alkyloxy-9 pyrido[4,3-b]carbazole (formule 10 sur le schéma réactionnel).

*Onzième étape.*

Dans cette étape, on traite le chloro-1 méthyl-5 alkyloxy-9 pyrido[4,3-b]carbazole obtenu à la dixième étape, de formule:

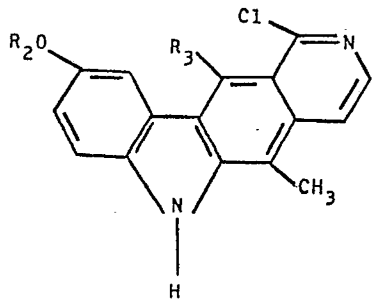

10

par une amine de formule $NH_2$—$R_1$, où $R_1$, $R_2$ et $R_3$ ont la signification indiquée précédemment. La substitution par l'amine se fait avantageusement à chaud, notamment à des températures de 100—200°C, et en particulier dans l'amine au reflux. L'invention concerne également ces composés de formule 10 à titre de nouveaux produits.

Les sels pharmaceutiquement acceptables desdits dérivés de l'invention peuvent être obtenus selon des moyens classiques à la portée de l'homme de l'art en utilisant des acides appropriés, tels que les acides chlorhydrique, bromhydrique, succinique, lactique, acétique maléique, phosphorique, et tous autres acides communément utilisés pour former de tels sels.

Les exemples ci-après illustrent, à l'aide d'un certain nombre de composés représentatifs, la synthèse selon l'invention. En référence à la figure annexée, on indiquera tout d'abord le schéma réactionnel utilisé dans les divers exemples.

Les chlorures de méthoxy-4 phényl-diazonium et benzyloxy-4 phényl-diazonium réagissent à 0°C avec le N-morpholino-1 cyclohexène et le méthyl-4-N-morpholino-1 cyclohexène en donnant respectivement les monoarylhydrazones de la cyclohexane dione-1,2 et de la méthyl-4 cyclohexane dione-1,2 (composés 1*a*, 1*b*, 1*c*, 1*d*).

En partant de ces arylhydrazones 1 (*a*—*d*), on a préparé successivement:

— les oxo-1 tétrahydro-1,2,3,4 alkyloxy-6 carbazoles *2* (*a*—*d*) qui résultent de la transformation des précédents par indolisation selon Fisher par la technique décrite par F. LIONS J. Proc. Roy. Soc. N.S. Wales *66* p. 516 (1933),

— les oxo-1 hydroxyméthylène-2 tétrahydro-1,2,3,4-alkyloxy-6 carbazoles *3* obtenus par acylation de *2* avec le formiate d'éthyle en présence d'hydrure de sodium, selon la technique décrite par E. WENKERT et K.G. DAVE J. Am. Chem. Soc. (1962), 84, p. 94.

— les oxo-1 isopropyloxyméthylène-2 tétrahydro-1,2,3,4-alkyloxy-6 carbazoles *4* formés en éthérifiant *3* avec l'iodure d'isopropyle en présence de carbonate de potassium dans le diméthylformamide, selon une technique semblable à celle décrite par Wenkert et Dave (cités supra).

— les méthyl-1 formyl-2 dihydro-3,4 alkyloxy-6 carbazoles *5* issus de la transformation de *4* par quatre équivalents molaires de méthyl-lithium suivie d'hydrolyse en milieu acide,

— less méthyl-1 formyl-2 alkyloxy-6 carbazoles *6* provenant de l'aromatisation de *5* au moyen du dioxyde de manganèse.

A partir des aldéhydes *6*, l'édification du cycle D des pyrido[4,3-b]carbazoles a été réalisée en trois étapes, dans les conditions de la réaction décrite par E. ELOY et A. DERYCKERE Helv. Chem. Acta. (1969), *52* p. 1755. Ainsi, l'acide malonique réagit avec *6 (a—d)* pour donner les acides acryliques *7 (a—d)* que l'on transforme en azides correspondants *8 (a—d)* par la méthode des anhydrides mixtes (11) et, dans le diphényl éther bouillant (10); ces derniers engendrent les dihydro 1,2 oxo-1 pyrido[4,3-b]carbazoles *9 (a—d)* qui conduisent aisément aux chloro-1 méthyl-5 alkyloxy-9 pyrido[4,3-b]carbazoles *10 (a—d)* par réaction avec l'oxychlorure de phosphore bouillant.

Les chloro-1 méthyl-5 alcoxy-9 pyrido[4,3-b]carbazoles 10 *(a—d)* sont substitués par les amines primaires ou secondaires pour donner naissance aux dérivés attendus *11* à *28*.

Les deux dérivés benzyloxylés 10*b* et 10*d* ont été synthétisés en vue de l'étude comparative des effets biologiques des dérivés méthoxylés et hydroxylés. En effet, par hydrogénation de leurs dérivés de substitution *24* et *28* sur charbon palladié, ils ont été débenzylés quantitativement en *29* et *30*.

On a rassemblé dans le tableau I ci-après les indications relatives à la signification de $R_1$, $R_2$ et $R_3$ dans les composés des exemples.

On notera que le composé *21* n'entre pas dans la définition générale des produits de l'invention. Les essais pharmacologiques ont en outre montré que ce composé n'est pas cytotoxique.

Dans les exemples qui suivent, les points de fusion, non corrigés, ont été pris sur un banc chauffant de Kofler ou avec un microscope à platine chauffante. Les spectres IR ont été enregistrés sur un spectrophotomètre Perkin Elmer à double faisceau, modèle 21. Sauf indications contraires, les spectres de RMN ont été enregistrés avec un appareil Hitachi-Perkin Elmer à 60 MHz, les autres l'ont été avec un

appareil Varian XL 100, avec le tétraméthylsilane comme référence interne et en solution dans $(CD_3)_2SO$.

Les composés affectés de l'indice *a* correspondent à $R_2 = CH_3$ et $R_3 = H$. Les composés affectés de l'indice *b* correspondent à $R_2 = CH_2—Ø$ et $R_3 = H$. Les composés affectés de l'indice *c* correspondent à $R_2 = R_3 = CH_2—Ø$ et $R_3 = CH_3$.

TABLEAU I

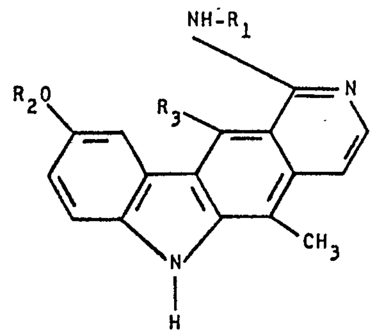

| | | | |
|---|---|---|---|
| 10*a* → | 11 | $R_2 = CH_3$; $R_3 = H$ | $R_1 = (CH_2)_2 - NH_2$ |
| | 12 | ,, ,, | $R_1 = (CH_2)_3 - NH_2$ |
| | 13 | ,, ,, | $R_1 = (CH_2)_4 - NH_2$ |
| | 14 | ,, ,, | $R_1 = (CH_2)_5 - NH_2$ |
| | 15 | ,, ,, | $R_1 = (CH_2)_6 - NH_2$ |
| | 16 | ,, ,, | $R_1 = (CH_2)_2 - N(CH_3)_2$ |
| | 17 | ,, ,, | $R_1 = (CH_2)_3 - N(CH_3)_2$ |
| | 18 | ,, ,, | $R_1 = (CH_2)_3 - N(C_2H_5)_2$ |
| | 19 | ,, ,, | $R_1 = -\underset{\mid \atop CH_3}{CH}-(CH_2)_3 - N(C_2H_5)_2$ |
| | 20 | ,, ,, | $R_1 = -(CH_2)_3 -$ 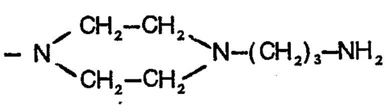 |
| | 21 | ,, ,, | $R_1 =$ 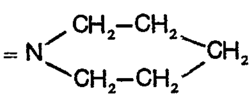 |
| 10*b* → | 22 | $R_2 = CH_2C_6H_5$; $R_3 = H$ | $R_1 = (CH_2)_2 - N(CH_3)_2$ |
| | 23 | ,, ,, | $R_1 = (CH_2)_3 - NH_2$ |
| | 24 | ,, ,, | $R_1 = (CH_2)_3 - N(C_2H_5)_2$ |
| 10*c* → | 25 | $R_2 = R_3 = CH_3$ | $R_1 = (CH_2)_3 - NH_2$ |
| | 26 | ,, ,, | $R_1 = (CH_2)_3 - N(CH_3)_2$ |
| | 27 | ,, ,, | $R_1 = (CH_2)_3 - N(C_2H_5)_2$ |
| 10*d* → | 28 | $R_2 = CH_2C_6H_5$; $R_3 = CH_3$ | $R_1 = (CH_2)_3 - N(C_2H_5)_2$ |
| 24 → | 29 | $R_2 = R_3 = H$ | $R_1 = (CH_2)_3 - N(C_2H_5)_2$ |
| 28 → | 30 | $R_2 = H$; $R_3 = CH_3$ | $R_1 = (CH_2)_3 - N(C_2H_5)_2$ |

Exemple 1

*(Méthoxy-4 phényl) hydrazones: composés 1a et 1c.*

On mélange 1a p. anisidine (123 g—1 mole) avec l'acide chlorhydrique concentré (172 ml—2 moles), on agite jusqu'à dissolution complète de 1a p. anisidine et on ajoute 400 g de glace. En maintenant la température de mélange en dessous de 5°C et en poursuivant l'agitation, on ajoute ensuite, goutte à goutte, une solution de nitrite de sodium (69 g—1 mole) dans le minimum d'eau. En continuant à agiter l'ensemble en-dessous de 3°C, on additionne alors l'énamine voulue (1 mole), correspondant respectivement à $R_2 = CH_3$ et $R_3 = H$ pour le composé 1*a* et $R_2 = CH_3$ et $R_3 = CH_3$ pour le composé 1*b*, en solution dans le dioxanne séché et diperoxydé (400 ml), on poursuit l'agitation pendant 1 heure en laissant revenir à la température ambiante, on essore le précipité et on le lave à l'eau puis à l'éthanol. Le solide rouge obtenu est repris dans 1 l d'éthanol bouillant et, après refroidissement, il est essoré et séché pour donner des cristaux rouge brique.

*(Benzyloxy-4 phényl) hydrazones: composés 1b et 1d.*

Le chlorhydrate de la benzyloxy-4 aniline finement pulvérisé (235,5 g—1 mole) est mis en suspension dans 500 ml d'acide chlorhydrique N; le mélange résultant est refroidi à 0°C puis traité successivement par le nitrite de sodium et l'énamine voulue (N-morpholino-1 cyclohexène ou méthyl-4-N-morpholino-1- cyclohexène) dans les mêmes conditions que pour former 1*a* et 1*b*.

Les caractéristiques des composés 1*a*, 1*b*, 1*c* et 1*d* sont données au tableau II.

TABLEAU II

Composés 1*a* — 1*b* — 1*c* — 1*d*

| | F°C (a) | Rdt % | Analyses c : calculé % t : trouvé % | | | |
|---|---|---|---|---|---|---|
| | | | | C | H | N |
| 1a+ | 204–5 déc. | 85 | c | 67,22 | 6,94 | 12,06 |
| | | | t | 67,28 | 6,82 | 12,02 |
| 1*b* | 161–2 | 76 | c | 74,00 | 6,54 | 9,09 |
| | | | t | 73,70 | 6,32 | 9,21 |
| 1*c* | 159–60 déc. | 77 | c | 68,27 | 7,37 | 11,37 |
| | | | t | 68,57 | 7,23 | 11,58 |
| 1*d* | 165 | 82 | c | 74,51 | 6,88 | 8,69 |
| | | | t | 74,38 | 6,65 | 8,74 |

\+ : Composé déjà décrit par V.I. Shvedov, L.B. Altukhova et A.N. Grinev Chemical Abstracts (1965) *63* p. 6893 h.

(a) : Echantillons analytiques recristallisés dans l'éthanol.

IR : $\nu$NH : 3220 à 3240, $\nu$C = 0 : 1660 à 1665, N = C : 1490 à 1495, 1515 à 1520 $cm^{-1}$.

Exemple 2

*Oxo-1 tétrahydro-1,2,3,4 alkyloxy-6 carbazoles: composés 2a, 2b, 2c et 2d.*

De l'acide sulfurique pur, d = 1,84 (196 g, 2 moles), d = 1,84 (196 g, 2 moles), est additionné à de l'éthanol absolu (2,5 l). En maintenant la solution chaude résultante sous agitation, on ajoute ensuite en une seule fois l'une des hydrazones obtenues à l'exemple 1 (1 mole). L'ensemble est chauffé à reflux pendant 2 à 4 heures, c'est-à-dire jusqu'à disparition totale de la tache correspondant au composé de départ sur plaque de gel de silice, et on laisse refroidir. Le précipité formé est assoré et les eaux-mères, concentrées à 800 ml et complétées à 2 l avec de l'eau, sont laissées sous agitation

pendant une nuit pour donner une petite fraction supplémentaire de la cétone attendue, à côte, d'une huile visqueuse noire. L'ensemble du solide est lavé à l'éthanol puis recristallisé dans le solvant indiqué dans le tableau III, qui indique également les caractéristiques des produits obtenus.

TABLEAU III

Composés 2*a* – 2*b* – 2*c* – 2*d*

| | F°C | Rdt % | Solvant de recristallisation | Analyses c : calculé % t : trouvé % | C | H | N |
|---|---|---|---|---|---|---|---|
| 2*a*+ | 216–19 | 60 | Ethanol | c | 72,54 | 6,09 | 6,51 |
| | | | | t | 72,34 | 6,22 | 6,51 |
| 2*b* | 211 | 40 | Diméthyl formamide | c | 78,33 | 5,88 | 4,81 |
| | | | | t | 78,05 | 5,93 | 4,76 |
| 2*c* | 133–4 Eb/0,15 = 214–40 | 30 | Ethanol | c | 73,34 | 6,60 | 6,11 |
| | | | | t | 73,49 | 6,56 | 6,24 |
| 2*d* | 193 | 50 | Xylène | c | 78,66 | 6,27 | 4,59 |
| | | | | t | 78,88 | 6,29 | 4,35 |

+ : Composé déjà décrit par B. Douglas, J. L. Kirkpatrick, B. P. Moore et J. A. Weisbach, Australian Journal of Chemistry (1964) *17*, 246.

IR : $\nu$NH : 3220 à 3260, $\nu$C = 0 : 1630 à 1640 $cm^{-1}$.

Exemple 3

*Oxo-1 hydroxyméthylène-2-tétrahydro-1,2,3,4 alkyloxy-6 carbazoles: composés 3a, 3b, 3c et 3d.*

Dans un tricol de 4 l muni d'uni réfrigérant et d'un système d'agitation, on introduit l'un des composés 2 obtenus à l'exemple 2 (1 mole) et le formiate d'éthyle (1300 ml — large excès), puis on ajoute progressivement l'hydrure de sodium à 50% dans l'huile (48 g—2 moles). Il se produit une réaction exothermique qui amène le formiate d'éthyle au reflux et, après 30 minutes sous agitation, on ajoute à nouveau 48 g d'hydrure de sodium et on continue la réaction pendant encore 30 minutes. Le mélange réactionnel refroidi est versé dans 2,5 l d'eau glacée; le mélange résultant est acidifié par l'acide chlorhydrique. Le précipité formé est filtré, lavé à l'eau, séché et recristallisé dans le solvant indiqué au tableau IV pour donner des cristaux jaunes. Les caractéristiques les produits 3 sont indiqués au tableau IV.

TABLEAU IV

Composés 3a – 3b – 3c et 3d

| | F°C | Rdt % | Solvant de recristallisation | Analyses<br>C : calculé %<br>t : trouvé % | C | H | N |
|---|---|---|---|---|---|---|---|
| 3a | 165–170 | 85 | Méthanol/eau 60/40 v/v | c | 69,16 | 5,39 | 5.76 |
| | | | | t | 69,39 | 5,46 | 5,48 |
| 3b | 190 | 95 | Acide acétique | c | 75,22 | 5,37 | 4,38 |
| | | | | t | 75,50 | 5,50 | 4,12 |
| 3c | 175–7 | 86 | Benzène | c | 70,00 | 5,88 | 5,44 |
| | | | | t | 70,13 | 5,70 | 5,29 |
| 3d | 178–82 dec. | 77,5 | Xylène | c | 75,65 | 5,74 | 4,20 |
| | | | | t | 75,82 | 5,96 | 3,92 |

IR : $\nu$OH : 3200 à 3280, $\delta$OH : 1110, $\nu$C = O : 1625 à 1635, $\nu$C = CHOH : 1565 à 1586, $\nu$C–O : 1250 à 1275 et 1325 à 1335 $cm^{-1}$.

Exemple 4

*Oxo-1 isopropyloxy méthyléne-2-tétrahydro-1,2,3,4 alkyloxy-6 carbazoles: composés* 4*a*, 4*b*, 4*c*, 4*d*.

Dans un tricol de 4 l on introduit l'un des dérivés hydroxyméthyléniques obtenus à l'exemple 3 (1 mole) le diméthylformamide fraichement rectifié (960 ml) et le carbonate de potassium sec (498 g—3,5 moles).

A ce mélange maintenu sous agitation et refroidi en dessous de 5°C par un bain de glace, on ajoute progressivement l'iodure d'isopropyle (840 g—5 moles). Après la fin de l'addition, on continue l'agitation aux environs de 0°C pendant 6 heures et on laisse enfin revenir à la température ambiante. Le précipité est essoré, lavé à l'acetone et le filtrat est évaporé. Le résidu de l'évaporation et le solide filtrés sont repris séparément dans l'eau, les précipités formés sont essorés et réunis pour être recristallisés dans le solvant indiqué dans le tableau V. Les éthers isopropyliques attendus se présentent sous la forme de paillettes ou aiguilles jaunes. Leurs caractéristiques sont indiquées au tableau V.

TABLEAU V

Ethers isopropyliques 4*a* – 4*b* – 4*c* et 4*d*

| | F°C | Rdt % | Solvant de recristallisation | Analyses c : calculé % t : trouvé % | | | |
|---|---|---|---|---|---|---|---|
| | | | | | C | H | N |
| 4*a* | 187–95 | 84 | Ethanol | c | 71,56 | 6,71 | 4,91 |
| | | | | t | 71,75 | 6,94 | 4,75 |
| 4*b* | 198 | 60 | Xylène | c | 76,43 | 6,41 | 3,88 |
| | | | | t | 76,14 | 6,62 | 3,97 |
| 4*c* | 167 | 67 | Méthanol ou cyclohexane | c | 72,20 | 7,07 | 4,68 |
| | | | | t | 71,96 | 6,88 | 4,67 |
| 4*d* | 146–55 | 60 | Cyclohexane | c | 76,72 | 6,71 | 3.73 |
| | | | | t | 76,95 | 6,72 | 3,41 |

IR : $\nu$C = O : 1650 à 1655, $\nu$CH–O : 1570 à 1580 $cm^{-1}$ .

RMN : DMSO d6 : 1,25 à 1,4 d ($CH_3$, J $CH_3$–CH = 6 Hz); 2,1 à 2,4 massif (C*H*); 4,3 à 4,45 multiplet (C*H*, J = 6 Hz).

Exemple 5

*Méthyl-1 formyl-2 dihydro-3,4 alkyloxy-6 carbazoles: composés* 5*a*, 5*b*, 5*c*, 5*d*.

L'un des éthers isopropyliques obtenus à l'exemple 4 (0,175 mole) placé dans un tricol de 1 l protégé de l'humidité est dissous dans l'éther anhydre (600 ml). A ce mélange refroidi par un bain de glace et maintenu sous agitation, on ajoute progressivement le méthyl-lithium (538 ml d'une solution éthérée dosée à 1,3M, soit 0,7 mole). La coloration rouge foncé observée au début disparaît vers la fin de l'addition et, après 30 minutes supplémentaires d'agitation, le mélange réactionnel est versé dans une solution saturée de chlorure d'ammonium (2 l). Le mélange résultant est extrait á l'éther en plusieurs fois et les couches organiques réunies sont agitées pendant 15 minutes en présence de 200 ml d'acide chlorhydrique 6N, ce qui provoque la formation d'un précipité brun-noir. On ajoute ensuite une solution d'hydroxyde de sodium jusqu'à pH alcalin, le précipité se redissout en grande partie et l'ensemble est extrait à l'éther ou au chloroforme, jusqu'à épuisement. L'ensemble de la couche organique est lavé avec une solution de soude N, puis à l'eau. Aprés séchage sur carbonate de potassium, le solvant est évaporé pour donner un résidu solide. Dans le cas des dérivés 5*a*, 5*b* et 5*c*, ce solide est repris dans le minimum de benzène, essoré, puis recristallisé dans le solvant indiqué au tableau VI.

En ce qui concerne le dérivé 5*d*, il a été purifié par chromatographie sur colonne de gel de silice,

avec le chlorure de méthylène comme solvant d'élution et en suivant l'évolution de la chromatographie sur plaques. Après évaporation des fractions contenant l'aldéhyde attendu presque pur, le résidu est recristallisé. Les composés 5 se présentent sous la forme de cristaux jaunes. Leurs caractéristiques sont indiquées au tableau VI.

Exemple 6

*Méthyl-1 formyl-2 alkyloxy-6 carbazoles: composés* 6*a*, 6*b*, 6*c*, 6*d*.

Dans un tricol de 4 l muni d'un dispositif d'agitation mécanique et d'un réfrigérant, on introduit l'un des dihydrocarbazoles 5 obtenus à l'exemple 5 (0,1 mole), le benzène sec (2 l) et on chauffe à l'ébullition pour homogénéiser. On ajoute ensuite, en une seule fois, le dioxyde de manganèse actif (15) (110 g—1,26 mole), on chauffe à reflux pendant 30 minutes en maintenant sous agitation et on filtre sous hotte. Le dioxyde de manganèse est lavé jusqu'à épuisement (acétone ou chloroforme selon les cas, au besoin à chaud). L'ensemble du filtrat est évaporé à sec et le résidu est recristallisé pour donner des aiguilles ou prismes jaunes. Les caractéristiques des composés 6 sont indiquées au tableau VII.

Exemple 7

*Acides trans-β[(méthyl-2 alkyloxy-6 carbazolyl)-2]acryliques: composés* 7*a*—7*b*—7*c* et 7*d*.

L'un des aldéhydes 6 obtenus à l'exemple 6 est dissous dans la pyridine sèche (800 ml) contenant de la pipéridine (3 ml) et l'ensemble est chauffé au reflux. A ce mélange, on ajoute de l'acide malonique (22,9 g—0,22 mole), on continue le chauffage au reflux pendant 15 minutes et on répète encore deux fois ce traitement par l'acide malonique, dont le total ajouté est donc de 0,66 mole. Après évaporation du solvant, le résidu solide est repris dans l'eau, essoré, lavé à l'eau et à l'acétone, séché et recristallisé ou simplement "digéré"

TABLEAU VI

Méthyl-1-formyl-2-dihydro-3,4-alkyloxy-6 carbazoles 5*a* – 5*b* – 5*c* – 5*d*

| | F°C | Rdt % | Solvant de recristallisation | RMN/DMSO d6 | | | | Analyses c : calculé % t : trouvé % | | | |
|---|---|---|---|---|---|---|---|---|---|---|---|
| | | | | $CH_3$–1 | $CHO$–2 | $CH_{3-4}$ | $NH$–9 | | C | H | N |
| 5*a* | 194 | 80 | Xylène | 2,5 | 10,6 | – | 8,6 | c | 74,66 | 6,27 | 5,81 |
| | | | | | | | | t | 74,72 | 6,18 | 5,85 |
| 5*b* | 158 | 76 | Benzène | 2,55 | 10,15 | – | 11,3 | c | 80,89 | 6,17 | 3,93 |
| | | | | | | | | t | 80,85 | 6,49 | 3,97 |
| 5*c* | 208 | 86 | Xylène | 2,5 | 10,5 | d 1,25 J = 6,5 Hz | 8,4 | c | 75,27 | 6,71 | 5,49 |
| | | | | | | | | t | 75,58 | 6,79 | 5,29 |
| 5*d* | 78–80 | 96,5 | Benzène | 2,45 | 10,6 | d 1,2 J = 6,5 Hz | 8,7 | c | 81,08 ≠ | 6,48 | 3,78 |
| | | | | | | | | t | 80,90 | 6,33 | 3,51 |

≠ calculé pour $C_{21}H_{19}NO_2$, 1/2 $C_6H_6$

IR : $\nu$C – O : 1605 à 1620 $cm^{-1}$,

TABLEAU VII

Méthyl-1 formyl-2 alkyloxy-6 carbazoles : *6a* – *6b* – *6c* et *6d*

| | F°C | Rdt % | Solvant de recristallisation | RMN/DMSO d6 | | | | Analyses c : calculé % t : trouvé % | | | |
|---|---|---|---|---|---|---|---|---|---|---|---|
| | | | | $CH_3$–1 | $CHO$–2 | $CH_{3-4}$ | $NH$–9 | | C | H | N |
| *6a* | 174 | 70 | Ethanol ou benzène | 2,9 | 10,4 | – | 11,3 | c | 75,30 | 5,40 | 5,85 |
| | | | | | | | | t | 75,37 | 5,65 | 5,96 |
| *6b* | 205 | 73 | Xylène | 2,9 | 10,5 | – | 11,5 | c | 79,88 | 4,99 | 4,44 |
| | | | | | | | | t | 79,92 | 5,32 | 4,71 |
| *6c* | | 88 | Benzène/cyclo-hexane l/l v/v | 2,8 | 10,45 | 2,8 | 10,15 | c | | | |
| | | | | | | | | t | | | |
| *6d* | 162–4 | 50 | Xylène | 2,85 | 10,8 | 2,8 | 11,8 | c | 81,67 ≠ | 6,28 | 3,66 |
| | | | | | | | | t | 81,65 | 6,03 | 3,55 |

IR : $\nu C-O$ : 1650 à 1675 $cm^{-1}$.

≠ calculé pour $C_{22}H_{19}NO_2$, 1/2 $C_8H_{10}$

dans l'acide acétique pour donner des microcristaux jaunes. Les caractéristiques des composés 7 sont indiquées au tableau VIII.

TABLEAU VIII

Acides acryliques 7*a* — 7*b* — 7*c* et 7*d*

| | F°C | Rdt % | RMN/DMSO d6 ╲CH = CH╲COOH (J = 16Hz) | Analyses c : calculé % t : trouvé % | C | H | N |
|---|---|---|---|---|---|---|---|
| 7*a* | 293–5 déc | 90 | 6,5 – 8,15 | c | 70,33 | 5,56 | 4,83[a] |
| | | | | t | 70,43 | 5,31 | 4,82 |
| 7*b* | 265–6 | 88,5 | 6,55 – 8,2 | c | 77,29 | 5,36 | 3,92 |
| | | | | t | 77,26 | 5,07 | 3,83 |
| 7*c* | 288–98 déc | 66 | 6,6 – 8,15 | c | 73,20 | 5,80 | 4,74 |
| | | | | t | 72,88 | 5,78 | 4,34 |
| 7*d* | 258–64 déc | 75 | 6,5 – 8,1 | c | 77,6 | 5,70 | 3,77 |
| | | | | t | 77,37 | 5,50 | 3,49 |

(a) Analyse calculée pour $C_{17}H_{15}NO_3$, 1/2 $H_2O$.

IR : $\nu$OH : 3400, bande large de 2200 à 3200, $\nu$C = O : 1660 à 1665; $\delta$OH : 930 à 940 $cm^{-1}$.

Exemple 8

*Trans-β[(méthyl-2 alkyloxy-6 carbazolyl)-2]-acrylazides: composés* 8*a*—8*b*—8*c et* 8*d*.

Le mélange constitué par l'un des acides acryliques 7 obtenus à l'exemple 7 (0,1 mole), la triéthylamine (11,1 g—0,11 mole) et l'acétone (260 ml) est refroidi à 0°C dans un bain de glace et de sel. Le chloroformiate d'éthyle (14,75 g—0,136 mole) en solution dans l'acétone (90 ml) est ensuite ajouté goutte à goutte, sous agitation et à 0°C, et, 1 heure après la fin de l'addition, le mélange hétérogène résultant maintenu à 0°C est traité par l'azoture de sodium (9,75 g—0,15 mole dissous dans le minimum d'eau), lui-même additionné goutte à goutte à 0°C. Une heure après la fin de l'addition, le bain réfrigérant est enlevé, et, lorsque le mélange est revenu à la température ambiante, il est versé dans l'eau, essoré, lavé à l'eau puis avec un peu d'acétone. Après séchage, les azides ainsi formés se présentent sous la forme de microcristaux qui ont été employés dans la réaction suivante sans autre purification.

Exemple 9

*Dihydro-1,2 oxo-1 méthyl-5 alkyloxy-9 (6 H) pyrido[4,3-b]carbazoles: composés* 9*a*, 9*b*, 9*c et* 9*d*.

Dans un tricol muni d'une ampoule à addition, d'un thermomètre plongeant et d'une agitation mécanique, on introduit du diphényléther (180 ml) et de tributylamine (4,1 g—22 mMoles). A ce mélange chauffé à 240°C et constamment maintenu à cette température, l'un des azides 8 obtenus à l'exemple 8 (20 mmoles), dispersé dans le diphényl-éther chauffé à 50°C (50 ml), est ajouté progressivement, sous violente agitation. Après la fin de l'addition, l'ensemble est maintenu à 240—250°C pendant 20 minutes, environ la moitié du diphényl-éther est distillé sous pression réduite et le solide formé après refroidissement et addition de benzène (100 ml) est essoré. Les pyrido[4,3-b]carbazoles 9 sont ensuite recristallisés dans le solvant indiqué au tableau IX. Toutefois, dans le cas de 9*a*, la purification est facilitée par un traitement préalable avec une solution de potasse N bouillante qui permet d'éliminer une petite quantité de l'acide 7*a*. La présence de ce dernier s'explique par sa faible solubilité qui, dans les conditions expérimentales utilisées, est à l'origine de sa transformation incomplète en azide correspondant 8*a*. Les caractéristiques des produits 9 sont indiquées au tableau IX.

Exemple 10

*Chloro-1 méthyl-5 (6H) alkyloxy-9 pyrido[4,3-b]carbazoles: composés* 10*a*—10*b*—10*c et* 10*d.*

L'un des dihydro-1,2 oxo-1 méthyl-5 pyrido[4,3-b]carbazoles 9 obtenus à l'exemple 9 (20 mmoles), est mis en suspension dans l'oxychloure de phosphore (1 l) et l'ensemble est chauffé au reflux, sous agitation. Généralement, il y a dissolution, précipitation d'un solide et finalement redissolution de ce dernier. Après le temps de reflux indiqué au tableau X, l'excès d'oxychlorure est éliminé sous pression réduite et le solide résiduel est repris dans l'eau, en présence de chloroforme.

A ce mélange maintenu sous agitation à la température ambiante, on ajoute une solution de soude N jusqu'à pH alcalin et on agite jusqu'à disparition de la coloration rouge et persistance d'un coloration jaune. Le précipité est essoré, la phase chloroformique est évaporée, le résidu est joint au précipité et le tout est recristallisé dans le solvant indiqué au tableau X. Celui-ci rassemble aussi les caractéristiques des composés 10.

## TABLEAU IX

Dihydro-1,2 oxo-1 méthyl-5 alkyloxy-9 (6H) pyrido [4,3-b] carbazoles *9a*, *9b*, *9c* et *9d*

| | F°C | Rdt % | Solvant de recristallisation | RMN/DMSO d6 | | | | Analyses c : calculé % t : trouvé % | | | |
|---|---|---|---|---|---|---|---|---|---|---|---|
| | | | | N*H*–2 | $CH_{3-5}$ | N*H*–9 | $CH_3$–11 | | C | H | N |
| *9a* | 297–302 | 47 | Acide acétique | 11,2 | 2,7 | 11,45 | – | c | 73,36 | 5,07 | 10,02 |
| | | | | | | | | t | 73,54 | 5,06 | 9,96 |
| *9b* | 258 | 72 | Dioxanne | 11,2 | 2,7 | 11,5 | – | c | 74,17 ≠ | 5,41 | 7,52 |
| | | | | | | | | t | 74,32 | 5,56 | 7,39 |
| *9c* | 292–8 | 59 | Dioxanne | 11,15 | 2,6 | 11,6 | 3,5 | c | 73,95 | 5,52 | 9,58 |
| | | | | | | | | t | 73,55 | 5,51 | 9,22 |
| *9d* | 268–70 | 30 | Dioxanne | 10,6 | 2,6 | 11,1 | 2,6 | c | 76,37≠≠ | 5,61 | 7,42 |
| | | | | | | | | t | 76,67 | 5,58 | 7,45 |

IR : $\nu$NH et OH : 2800 – 3350; $\nu$C=O : 1635 à 1650 $cm^{-1}$

≠ Calculé pour $C_{23}H_{18}N_2O_2$, $H_2O$ ≠≠ Calculé pour $C_{24}H_{20}N_2O_2$, 1/2 $H_2O$.

TABLEAU X

Chloro-1 méthyl-5 alkyloxy-9 pyrido [4,3-b] carbazoles
10*a* – 10*b* – 10*c* et 10*d*

| | F°C | Rdt % | Solvant de recristallisation | Analyses c : calculé % t : trouvé % | C | H | N | Cl |
|---|---|---|---|---|---|---|---|---|
| 10*a* | 264 | 73 | Acétate d'éthyle | c | 68,80 | 4,38 | 9,44 | 11,97 |
| | | | | t | 68,52 | 4,40 | 9,25 | 11,84 |
| 10*b* | 248–50 | 88 | Ethanol puis xylène | c | 72,82≠ | 5,05 | 7,08 | 8,97 |
| | | | | t | 72,70 | 4,82 | 6,96 | 9,11 |
| 10*c* | 241–5 | 25 | Xylène | c | 69,56 | 4,83 | 9,02 | 11,43 |
| | | | | t | 69,80 | 5,07 | 9,28 | 11,68 |
| 10*d* | 221–2 | 70 | Xylène | c | 74,51 | 4,91 | 7,24 | 9,18 |
| | | | | t | 74,67 | 5,12 | 7,32 | 9,02 |

≠ Calculé pour $C_{23}H_{17}$ Cl $N_2O$, 1/2 $C_2$ $H_5$ OH.

Exemples 11 à 28

*Amino alkylamino-1 méthyl-5 alkyloxy-9 pyrido[4,3-b]carbazoles: composés 11 à 28.*

A l'un des composés chlorés 10 obtenus à l'exemple 10 (500 mg), on ajoute 8 ml ou 8 g de l'amine voulue de formule $NH_2$—$R_1$ et ce mélange est chauffé à la température et pendant les temps indiqués au tableau XI. Après évaporation de l'excès d'amine sous vide variant de 15 à 0,5 mm de Hg suivant les cas, le résidu est repris dans 100 ml d'une solution d'hydroxyde de sodium 0,5 N et le solide obtenu est essoré, séché puis recristallisé. Les conditions de la réaction et les caractéristiques des composés de l'invention sont indiquées au tableau XI.

On a préparé le bimaléate du composé 27 selon le mode opératoire ci-après.

On a dissous le composé 27 dans de l'alcool. Séparément on a dissous dans de l'alcool 2 quantitiés équimolaires d'acide maléique avec un excès de 10%. On a mélangé les deux solutions. On a porté à l'ébullition quelques instants puis on a laissé refroidir. Le composé obtenu, c'est-à-dire le composé qui cristallise est le bimaléate du composé 27, dont le point de fusion est environ 170°C.

Exemples 29 et 30

*γ-diéthylamino-propylamino-1-méthyl-5(et diméthyl-5,11)-hydroxy-9 pyrido[4,3-b]carbazoles: composés 29 et 30, respectivement.*

Au composé benzyloxylé 24 ou 28 (1mmole) dissous dans 100 ml d'éthanol maintenu dans un bain-marie chauffé à 50—55°C, on ajoute 40 mg de charbon palladié à 30% et on agite sous atmosphère d'hydrogène à la pression normale pendant 4 heures. Le catalyseur est filtré, le solvant évaporé et le résidu recristallisé dans le xylène en donnant des microcristaux jaune clair (tableau XI).

Exemple 31

Le mélange constitué par le dihydro-1,2 oxo-1 méthyl-5 méthoxy-9 pyrido[4,3-b]carbazole 9*a* (5 g—18 mmoles) et le chlorhydrate de pyridine (50 g) est chauffé à 220—225°C pendant 30 minutes et versé dans l'eau glacée. Le précipité formé est essoré, lavé à l'eau et recristallisé dans l'éthanol en présence de charbon actif pour donner 2,6 g (55%) de microcristaux beiges, infusibles à 350°C.

Analyse: calculé % pour $C_{16}$ $H_{12}$ $N_2$ $O_2$:
C: 72,71 H 4,38 N 10,60
trouvé % 72,57 4,34 10,49

L'invention procure donc des produits de la famille des ellipticines portant divers substituants, aussi bien sur leur cycle A que sur leurs sommes 11 et 1. Les nouveaux composés présentent des activités cytotoxiques sur cellules en culture et une activité antitumorale sur leucémie L 1210 très prononcées. Les composés selon l'invention possèdent également un pouvoir protecteur sur la leucémie viro-induite de Friend (J. Exp. Méd. 1957 — *105,* 307—318); ce sont donce également des agents antiviraux et antitumoraux.

*Essais pharmacologiques.*

Les composés selon l'invention testés dans les essais ci-après ont été utilisés sous la forme de solutions aqueuses préparées en présence d'une quantité suffisante d'acide pour solubiliser le composé considéré, sauf stipulation contraire.

*Essai 1:* Etude des propriétés antitumorales des composés de l'invention sur la leucémie L 1210.

*A — Mesure du temps de survie.*

Les propriétés antitumorales ont été déterminées d'après l'action curative sur la leucémie expérimentale greffée L 1210. Cette leucémie a été entretenue sur des souris $B_6D_2$Fl (C 57 B1/6 x DBA/2) Fl à raison de six ou dix souris par lot expérimental. Les composés a tester ont été injectés par voie intrapéritonéale un ou plusieurs jours après la greffe des cellules (1 seule injection). Les résultats obtenus sont rassemblés dans le tableau XII. On a déterminé l'intervalle de mort, le premier chiffre du tableau indiquant la date de la première souris morte et le second la date de la dernière souris morte. On a également mesuré la moyenne du temps de survie (MST). Une valeur caractéristique est le pourcentage d'augmentation du temps de survie (ILS %). Cette valeur (Cancer. Res. 1971, *31,* 1883—1887) est le rapport:

$$ILS\,\% = \frac{S^t - S^c}{S^c} \times 100$$

où $S^t$ = survie des animaux traités et

$S^c$ = survie des animaux témoins.

Les résultants du tableau XII montrent que les produits selon l'invention possèdent des propriétés antitumorales.

TABLEAU XI

Substitution des chloro-1 méthyl-5 alkyloxy-9 pyrido [4,3-b] carbazoles
10*a* – 10*b* – 10*c* et 10*d* – Dérivés 11 à 30

| Produit de départ | Composé obtenu | F°C | Rdt % | Conditions de la réaction temps et température | Solvant de recristallisation |
|---|---|---|---|---|---|
| 10*a* | 11 | 180–185 | | 2 heures reflux | méthanol acétonitrile |
| – | 12 | 190 | 71 | 1 heure reflux | méthanol acétonitrile |
| – | 13 | 230–238 (déc) | 48 | 45 minutes reflux | méthanol acétonitrile |
| – | 14 | 115 env. | 82 | 45 minutes reflux | méthanol acétonitrile l/l v/v |
| – | 15 | 210–15 | 61,5 | 45 minutes reflux | méthanol acétonitrile l/l v/v |
| – | 16 | 235–40 | 63 | 19 heures reflux | éthanol acétonitrile l/l v/v |
| – | 17 | 195–200 | 84 | 7 heures reflux | éthanol acétonitrile l/l v/v |
| – | 18 | 185–7 | 23 | 2 heures reflux | benzène acétonitrile |
| – | 19 | 74–75 | 40 | 5 heures reflux/$N_2$ a l'obscurité | cyclohexane |
| – | 20 | 190–5 | 73,5 | 1 heure à 150°C | xylène |

TABLEAU XI (suite)

Substitution des chloro-1 méthyl-5 alkyloxy-9 pyrido [4,3-b] carbazoles
10*a* – 10*b* – 10*c* et 10*d* – Dérivés 11 à 30

| Produit de départ | Composé obtenu | Formule brute du composé analysé (solvant associé) | Analyses<br>c : calculé %<br>t : trouvé % | C | H | N |
|---|---|---|---|---|---|---|
| 10*a* | 11 | $C_{19}H_{20}N_4O$ (1/2 $N_2O$) | c<br>t | 69,30<br>68,88 | 6,38<br>6,29 | 17,02<br>17,13 |
| – | 12 | $C_{20}H_{22}N_4O$ ($CH_3$ CN) | c<br>t | 70,37<br>70,21 | 6,71<br>6,69 | 18,65<br>18,80 |
| – | 13 | $C_{21}H_{24}N_4O$ (1/2 $CH_3CH$, 1$H_2O$) | c<br>t | 70,12<br>69,96 | 7,30<br>6,98 | 16,73<br>16,76 |
| – | 14 | $C_{22}H_{26}N_4O$ (1/2 $CH_3CN$, 3$H_2O$) | c<br>t | 67,39<br>67,76 | 7,45<br>7,35 | 15,38<br>15,57 |
| – | 15 | $C_{23}H_{28}N_4O$ (1/2 $H_2O$) | c<br>t | 71,68<br>71,50 | 7,27<br>7,57 | 14,54<br>14,63 |
| – | 16 | $C_{21}H_{24}N_4O$ (1/2 $H_2O$) | c<br>t | 70,59<br>70,69 | 7,00<br>6,87 | 15,68<br>15,57 |

TABLEAU XI (suite)

Substitution des chloro-1 méthyl-5 alkyloxy-9 pyrido [4,3-b] carbazoles
10*a* – 10*b* – 10*c* et 10*d* – Dérivés 11 à 30

| Produit de départ | Composé obtenu | Formule brute du composé analysé (solvant associé) | Analyses c : calculé % t : trouvé % | C | H | N |
|---|---|---|---|---|---|---|
| 10*a* | 17 | $C_{22}H_{26}N_4O$ ($H_2O$) | c<br>t | 69,47<br>69,74 | 7,36<br>7,41 | 14,73<br>13,97 |
| – | 18 | $C_{24}H_{30}N_4O$ ($CH_3CN$) | c<br>t | 72,36<br>72,72 | 7,71<br>7,73 | 16,23<br>15,80 |
| – | 19 | $C_{26}H_{34}N_4O$ ($H_2O$) | c<br>t | 71,52<br>71,33 | 8,31<br>8,32 | 12,83<br>12,45 |
| – | 20 | $C_{27}H_{36}N_6O$ (1/4 $C_8H_{10}$) | c<br>t | 71,53<br>71,55 | 7,91<br>7,92 | 17,26<br>17,46 |
| – | 21 | $C_{22}H_{23}N_3O$ | c<br>t | 76,49<br>76,43 | 6,71<br>6,91 | 12,17<br>12,11 |
| 10*b* | 22 | $C_{27}H_{28}N_4O$ | c<br>t | 76,38<br>75,67 | 6,65<br>6,62 | 13,20<br>12,70 |
| – | 23 | $C_{26}H_{26}N_4O$ (1/2 $C_2H_5OH$) | c<br>t | 74.82<br>75,77 | 6,69<br>6,59 | 12,93<br>12,49 |
| – | 24 | $C_{30}H_{34}N_4O$ | c<br>t | 77,22<br>76,96 | 7,35<br>7,33 | 12,01<br>11,73 |

TABLEAU XI (suite)

Substitution des chloro-1 méthyl-5 alkyloxy-9 pyrido [4,3-b] carbazoles
10*a* – 10*b* – 10*c* et 10*d* – Dérivés 11 à 30

| Produit de départ | Composé obtenu | Formule brute du composé analysé (solvant associé) | Analyses c : calculé % t : trouvé % | | | |
|---|---|---|---|---|---|---|
| | | | | C | H | N |
| 10*c* | 25 | $C_{21}H_{24}N_4O$ ($C_2H_5OH$, 2 $H_2O$) | c<br>t | 64,16<br>63,83 | 7,96<br>7,56 | 13,01<br>12,99 |
| – | 26 | $C_{23}H_{28}N_4O$ ($C_2H_5$ OH) | c<br>t | 71,06<br>70,85 | 8,11<br>8,21 | 13,26<br>13,11 |
| – | 27 | $C_{25}H_{32}N_4O$ | c<br>t | 74,22<br>73,98 | 7,97<br>7,92 | 13,85<br>13,94 |
| 10*d* | 28 | $C_{31}H_{36}N_4O$ | c<br>t | 77,46<br>77,19 | 7,55<br>7,38 | 11,66<br>11,62 |
| 24 | 29 | $C_{23}H_{28}N_4O$ (1/2 $H_2O$) | c<br>t | 71,69<br>71,97 | 7,58<br>7,55 | 14,54<br>14,26 |
| 28 | 30 | $C_{24}H_{30}N_4O$ ($H_2O$) | c<br>t | 70,56<br>70,86 | 7,90<br>7,67 | 13,72<br>13,48 |

TABLEAU XI

Substitution des chloro-1 méthyl-5 alkyloxy-9 pyrido [4,3-b] carbazoles
10*a* – 10*b* – 10*c* et 10*d* – Dérivés 11 à 30

| Produit de départ | Composé obtenu | F°C | Rdt % | Conditions de la réaction temps et température | Solvant de recristallisation |
|---|---|---|---|---|---|
| 10*a* | 21 | 220 | 64 | 15 heures reflux | éthanol |
| 10*b* | 22 | 228 | 54 | 22 heures reflux | cyclohexane benzène 1/1 v/v |
| – | 23 | 230–2 | 45 | 1 heure 20 reflux | xylène |
| – | 24 | 207–14 | 78,5 | 2 heures 30 reflux | éthanol |
| 10*c* | 25 | 210 | 38 | 30 minutes reflux | benzène |
| – | 26 | 227 | 51,5 | 6 heures reflux | éthanol |
| – | 27 | 156 | 47 | 1 heure reflux | cyclohexane |
| 10*d* | 28 | 175 | 50 | 1 heure 30 reflux | xylène |
| 24 | 29 | 218–24 | 85 | | xylène |
| 28 | 30 | 125 | 62 | | xylène |

TABLEAU XII

Effet des composés de l'invention sir la leucémie L1210 *in vivo*

| | Composé No | Dose mg/kg | Jour d'inocu-lation | Intervalle de mort (souris) | MST | ILS % | Souris survivantes |
|---|---|---|---|---|---|---|---|
| $10^5$ cellules | Témoins | – | – | 9 – 13 | 10,5 | | |
| | 9–MeO$^+$ | 87,5 | +1 | 13 – 15 | 13,3 | 26,6 | |
| | | 35 | +1 | 11 – 13 | 11,3 | 7,6 | |
| | | 17,5 | +1 | 10 – 13 | 11,2 | 6,6 | |
| $10^5$ cellules | Témoins | – | – | 10 – 13 | 11,6 | – | |
| | 9–MeO$^+$ | 20 | +2 | 15 – 21 | 17,6 | 51,7 | |
| | 9–MeO$^+$ | 20 | +3 | 18 – 26 | 20,9 | 80 | |
| $10^5$ cellules | 18 | 20 | +3 | 13 – 19 | 16.1 | 38,8 | – |
| | 19 | 20 | +4 | 11 – 16 | 12,4 | 19,2 | – |
| | 19 | 10 | +4 | 11 – 16 | 13 | 25 | – |
| | 29 | 20 | +4 | 10 – 15 | 12,4 | 19,2 | – |
| | 29 | 10 | +4 | 15 – 17 | 16,2 | 55,8 | – |

TABLEAU XII (suite)

Effet des composés de l'invention sur la leucémie L1210 *in vivo*

| | Composé No | Dose mg/kg | Jour d'inocu-lation | Intervalle de mort (souris) | MST | ILS % | Souris survivantes |
|---|---|---|---|---|---|---|---|
| $10^5$ cellules | 25 | 20 | +4 | 8 – 12 | 9,6 | – | – |
| | 25 | 10 | +4 | 12 – 13 | 12,6 | 21,1 | – |
| | 26 | 20 | +4 | 16 – 19 | 17,4 | 67,3 | – |
| | 26 | 10 | +4 | 12 – 16 | 14,4 | 38,5 | – |
| | 27 | 20 | +4 | 17 – 23 | 20,2 | 94,2 | — |
| | 27 | 10 | +4 | 16 – 27 | 20 | 92,3 | – |
| | 27 | 15 | +1 | 17 – 43 | 24 | 128,6 | – |
| | 27 | 20 | +3 | 10 – 30 | 16,5 | 42,7 | – |
| | 27 | 10 | +3 | 12 – 31 | 17,3 | 49,4 | – |
| ++ | 27 | 15 | +1 | 19 – 35 | 25 | 134 | |
| ++ | 27 | 20 | +1 | 20 – 31 | 25,5 | 102,4 | 4 |
| ++ | 27 | 15 | +1 | 20 – 32 | 26 | 104,7 | 4 |

+ : méthoxy-ellipticine

++ : lot expérimental de six souris.

Dans les essais rapportés ci-dessus, on a choisi comme composé de référence a 9-méthoxy elipticine utilisée en clinique humaine dans le traitement des leucémies myéloides aigües. Ce composé présente une augmentation du temps de survie (ILS) de 26% à la dose la plus proche de la dose toxique, alors, que dans les mêmes conditions, un composé de l'invention, tel que le composé 27, fournit une valeur ILS de 134%.

*B — Effet du jour d'inoculation.*

On a opéré sur des lots expérimentaux de six souris selon le mode opératoire défini précédemment, en injectant $10^5$ cellules au jour Jo et en utilisant le composé 27 selon l'invention et en faisant varier le jour d'inoculation du composé 27 d'un lot à l'autre. Le composé 27 a été inoculé à la dose de 15 mg/kg de souris. Comme dans l'essai ci-dessus on a déterminé l'intervalle de mort, mesuré la moyenne du temps de survie (MST) et calculé le pourcentage d'augmentation du temps de survie (ILS %) selon la formule définie ci-dessus. Les résultats obtenus sont rassemblés dans le tableau XIII ci-après.

TABLEAU XIII

Effet du jour d'inoculation du composé 27

| | Jour d'inoculation | Intervalle de mort | Moyenne du temps de survie | ILS % | Survivantes |
|---|---|---|---|---|---|
| Témoin | – | 10 – 14 | 11,5 | 0 | 0 |
| Composé 27 | Jo(+3h) | 16 – 25 | 20,7 | 80 | 0 |
| ,, | J+1 | 15 – 29 | 21 | 82,6 | 2 |
| ,, | J+2 | 15 – 35 | 21,7 | 88,7 | 0 |
| ,, | J+3 | 13 – 25 | 18 | 56,5 | 0 |
| ,, | J+4 | 14 – 20 | 16 | 39,1 | 0 |

Les résultats ci-dessus montrent que le composé 27 est plus actif un jour après la greffe des cellules (2 souris survivantes sur 6 souris traitées); ceci signifie que le composé 27 est plus actif vis-à-vis des cellules en division. Au jour J+2 le composé 27 présente encore une forte activité (ILS % = 88,7) alors que le nombre de cellules a fortement augmenté, ce qui explique la baisse d'activité apparente du composé 27 aux jours J+3 et J+4 qui est due au nombre croissant de cellules tumorales dans le péritoine de l'animal.

*C— Relation dose/effet.*

On a opéré sur des lots expérimentaux de six souris selon le mode opératoire défini précédemment. On a injecté $10^5$ cellules au jour Jo; l'inoculation a été réalisée au jour J+1 avec le composé 27 à des doses de 5 mg/kg; 10 mg/kg; 15 mg/kg et 20 mg/kg. On a déterminé l'intervalle de mort, mesuré la moyenne du temps de survie (MST) et calculé le pourcentage d'augmentation du temps de survie (ILS %) selon la formule définie ci-dessus. Les résultats obtenus sont rapportés dans le tableau XIV ci-après:

TABLEAU XIV

Relation dose/effet

| | Dose mg/kg | Intervalle de mort | Moyenne du temps de survie | ILS % | Survivantes |
|---|---|---|---|---|---|
| Témoin | – | 10 – 12 | 10,7 | – | 0 |
| Composé 27 | 5 | 16 – 24 | 19,3 | 80,4 | 0 |
| ,, | 10 | 14 – 21 | 16,8 | 57 | 0 |
| ,, | 15 | 13 – 31 | 21 | 96,3 | 0 |
| ,, | 20 | 15 – 25 | 18,8 | 75,7 | 1 |

Les résultats du tableau XIV montrent que l'activité du composé 27 est proportionnelle à la dose inoculée. Si 1, ILS calculée pour la dose de 20 mg/kg parait inférieure à celle obtenue à la dose de 15 mg/kg c'est que dans le calcul n'entre pas la souris définitivement guérie.

*Essai 2:* Etude cytotoxique "in vitro" des composés de l'invention sur des cellules de la leucémie viro-induite de Friend de la souris (J. Exp. Med. 1957, *105,* p. 307—318).

L'activité antitumorale a été mesurée sur une lignée tumorale dérivée de la leucémie murine due au virus de C. Friend.

Les cellules tumorales se multiplient en suspension dans le milieu RPMI 1640 [catalogue de GIBCO Bio-Cult-Ltd, Washington Road Sandyford Industrial Estate Paisley PA3 4EP Renfrewshire Scotland] additionné de 20% de sérum de veau embryonnaire, de pénicilline et de streptomycine. Le temps de doublement de la culture est d'environ 11 heures. Le facteur de croissance est égal à 1 ou très voisin de 1 (toutes les cellules se multiplient).

Les cultures sont ensemencées au temps $t = 0$ à la concentration de $2 \times 10^5$ cellules par ml dans des boîtes Falcon contenant 4 ml de milieu. Vingt quatre heures plus tard, le produit à tester (en solution dans l'eau acétique) a été ajouté, au moment où les cellules sont en phase exponentielle de croissance. Vingt quatre heures après l'addition du produit, les cellules ont été dénombrées et le pourcentage de cellules vivantes déterminé par un test d'exclusion au bleu trypan.

On peut définir deux doses:

1) la dose léthale 100% (DL 100)

2) la dose léthale 50% (DL 50).

Les résultats sont indiqués dans le tableau XV.

TABLEAU XV

Etude cytotoxique "in vitro" des composés de l'invention sur des cellules de la leucémie de Friend de la souris

| | DL 100 (conc. molaire) | DL 50 | DL 50 HUM / DL 50 produit |
|---|---|---|---|
| Produit de référence : acétométhylate-hydroxy-9-ellipticinium (HUM) | 8 . $10^{-7}$ | 3 . $10^{-7}$ | 1 |
| Composés de l'invention | | | |
| 11 | 2,5 . $10^{-7}$ | $10^{-7}$ | 3 |
| 12 | 7 . $10^{-7}$ | 2,5 . $10^{-7}$ | 1,5 |
| 13 | 1,5 . $10^{-5}$ | 7 . $10^{-6}$ | 0,04 |
| 14 | 1,5 . $10^{-6}$ | 5 . $10^{-7}$ | 0,6 |
| 15 | 2 . $10^{-6}$ | 8 . $10^{-7}$ | 0,4 |
| 16 | 3 . $10^{-6}$ | 8 . $10^{-7}$ | 0,4 |
| 17 | 3 . $10^{-6}$ | 1,5 . $10^{-6}$ | 0,2 |
| 18 | 1,5 . $10^{-6}$ | 5 . $10^{-7}$ | 0,6 |
| 19 | 3 . $10^{-6}$ | 1,3 . $10^{-6}$ | 0,2 |
| 21 | $10^{-4}$ | 3,5 . $10^{-5}$ | 1,1 |
| 23 | 9 . $10^{-7}$ | 3 . $10^{-7}$ | 1 |
| 24 | . $10^{-5}$ | 3 . $10^{-6}$ | 0,1 |
| 25 | 3 . $10^{-7}$ | 1,2 . $10^{-7}$ | 3 |
| 26 | . $10^{-6}$ | 4 . $10^{-7}$ | 0,75 |
| 27 | 1,2 . $10^{-6}$ | 4,5 . $10^{-7}$ | 0,65 |
| 29 | 4 . $10^{-8}$ | 1,7 . $10^{-8}$ | 18 |

Ces résultats montrent que la plupart des produits sont cytotoxiques à des concentrations comprises entre 3 . $10^{-6}$ et 3 . $10^{-7}$M.

Trois composés (les composés 11, 25 et 29) sont plus actifs que les dérivés déjà connus, en particulier l'acétate de méthyl-2 hydroxy-9 ellipticinium servant de référence (HUM). Les autres composés sont pratiquement aussi actifs que l'HUM.

*Essai 3:* Etude cytotoxique "in vitro" sur des cellules de hamster et sur des cellules de la leucémie L 1210 de la série.

En particulier, on a utilisé la lignée BHK 21 de cellules de hamster et un clone dérivé de cette lignée, transformé par le virus du sarcome de hamster (clone HS5).

Après détachement par la trypsine, les cellules ont été mises en culture dans des boîtes de Petri en plastique de 35 mm de diamètre à la concentration de 2.$10^5$ cellules/boîte, dans un milieu de culture de "Eagle" additionné de "Bactotryptophosphate Broth Difco" et de 10% de sérum de veau [M. STOKER et I. MAC-PHERSON Virology, *14,* 1961, 359].

Après 5 heures, les cellules ont été attachées sur un support plastique et on a ajouté les produits à tester dont les solutions ont été faites dans l'eau ou dans le DMSO (diméthyl-sulfoxyde) si le produit est

peu soluble dans l'eau. Dans ce dernier cas, un témoin a été effectué avec la même concentration finale de DMSO dans le milieu de culture.

L'état des cellules a été examiné 24, 48 et 72 heures après.

Dans le cas des cellules L 1210, les essais ont été effectués en milieu gélifié par de l'agarose, les cellules L 1210 se multipliant en suspension.

Les résultats sont indiqués dans le tableau XVI.

Les résultats, (tableau XVI) montrent clairement que les produits, à l'exception du produit 21, sont nettement cytotoxiques à des concentrations comprises entre 0,4 et 5 $\mu$m, et que les produits les plus actifs, c'est-à-dire les composés 24 et 27, sont presque aussi actifs que le dipyrido-indole de référence (BD40).

Les effets inhibiteurs sur les deux types de cellules, normal et transformé, sont similaires.

On notera que le composé 21 n'entre pas dans le cadre de la formule générale des composés revendiqués.

TABLEAU XVI

Dose minimale entraînant une inhibition complète de la croissance

| | 11 | 12 | 13 | 14 | 15 | 16 | 17 | 18 | 21 | 24 | 25 | 27 | Référence BD 40+ |
|---|---|---|---|---|---|---|---|---|---|---|---|---|---|
| C13/8 | 1$\mu$M | 2 | 2 | 2 | 2 | 1 | 1 | 5 | >5 | | | | 0,25 |
| HS5 | 1 | 2 | 2 | 2 | 2 | 2 | 2 | 5 | >5 | | | | 0,25 |
| L1210 | | | | | | | | | | 0,4 | 0,9 | 0,6 | 0,2 |

+BD 40 = ($\gamma$-diéthylaminopropyl)-amino-1-méthyl-5-dipyrido [4,3-b] [3,4-f]indole.

*Essai 4:* Leucémie viro-induite: leucémie de Friend.

Les expériences ci-après ont portè sur le traitement des souris inoculées par le virus de la leucémie de Friend.

On a utilisé dans ces expériences des souris $DBA_2$, mâles, âgées de 2 à 3 mois, d'un poids voisin de 20 g.

On a utilisé une souche Anémiante du virus de Friend (VFA) entretenue depuis plusieurs années au laboratoire. Le stock viral est constitué d'un surnageant acellulaire obtenu par centrifugation d'un broyat de rates leucémiques, effectué dans une solution de PBS-saccharose 0,5 M. Le titrage du stock se fait in vivo selon la méthode décrite par JASMIN et al. [J. Nah. Cancer Inst. 1974, *53*, 469—474]. Le titre est exprimé en $SD_{50}$ (Spleen enlarging dose 50%).

*A — Mesure de la splénomégalie.*

*1 — Protocole d'essai*

Le virus de Friend est injecté à la souris par voie intrapéritonéale. L'animal développe alors une splénomégalie caractéristique. La mortalité apparaissant environ trois semaines après l'inoculation virale aux doses de virus employés habituellement, on a sacrifié les animaux le jour + 15 après l'infection, pour l'étude de la splénomégalie. Les rates sont prélevées puis pesées individuellement. Toute souris dont la rate avait un poids supérieur à 250 mg était considérée leucémique.

*2 — Résultats.*

Les résultats obtenus sont rassemblés dans le tableau XVII ci-après. Ils montrent que le composé 27 selon l'invention présente un pouvoir protecteur sur la leucémie de Friend.

B — Mesure du temps de survie

Dans une autre série d'essais on a suivi quotidiennement la mortalité des souris, auxquelles on a injecté le virus de Friend (UFA) par voie intrapéritonéale (dilution 1/800) et on a déterminé l'intervalle de mort, la moyenne du temps de survie et le pourcentage d'augmentation du temps de survie pour le composé 27 selon l'invention inoculé au jour J+1 à la dose de 15 mg/kg.

Les résultats obtenus sont rassemblés dans le tableau XVIII ci-après.

*C — Relation dose/effet*

On a opéré sur lots expérimentaux de 10 souris, auxquelles on a injecté le virus VFA (dilution 1/800-% de leucémies = 100%) au jour Jo. On a ensuite injecté le composé 27 selon l'invention au jour

J+1 á des doses différentes selon les lots et on a déterminé l'intervalle de mort, la moyenne du temps de survie et le pourcentage d'augmentation du temps de survie.

Les résultats obtenus sont rassemblés dans le tableau XIX ci-après. Ces résultats montrent que l'activité du composé 27 est proportionnelle à la dose inoculée.

TABLEAU XVII

Leucémie de Friend (mesure de la Splénomégalie)

| Virus inoculé au jour O | Dilution du virus | Composé essayé | Dose | Jour d'injection | Poids moyen de la rate (mg)$^{++}$ | Nombre de souris inoculées | Nombre$^{+}$ de souris leucémiques |
|---|---|---|---|---|---|---|---|
| VFA | 1/800 | PBS$^{+++}$ | 0,1 ml/souris | J+1 | 1.503<br>(2.074 – 1.055) | 10 | 10/10 |
| VFA | 1/800 | Composé 27 | 0,2 mg/souris<br>10 mg/kg | J+1 | 183<br>(269–123) | 10 | 2/10 |
| VFA | 1/6000 | PBS$^{+++}$ | 0,1 ml/souris | J+1 | 827<br>(1.977 – 158) | 10 | 7/10 |
| VFA | 1/1600 | Composé 27 | 0,2 mg/souris<br>10 mg/kg | J+1 | 199<br>(236 – 155) | 5 | 0/5 |
| VFA | 1/3200 | PBS$^{+++}$ | 0,1 ml/souris | J+1 | 477<br>(1.117–100) | 10 | 7/10 |
| VFA | 1/3200 | Composé 27 | 0,2 mg/souris<br>10 mg/kg | J+1 | 220<br>(280–191) | 5 | 1/5 |

+ = Sont considérées comme leucémiques, les souris dont le poids de la rate est supérieur à 250 mg.

++ = Entre parenthèses, la rate avec le poids le plus fort (1er chiffre) et celle avec le poids le plus faible (2ème chiffre).

+++ = Tampon phosphate.

TABLEAU XVIII

Essai sur le virus de Friend anémiant

| Dilution du virus VFA | Composé testé | Intervalle de mort | Moyenne du temps de survie | ILS % | survivantes |
|---|---|---|---|---|---|
| 1/800 % de leucémies = 99,8 | TEMOIN VIRUS 1 | 19 – 43 | 30,5 } 30,15 | – | 1 |
| | | 22 – 37 | 29,8 } | – | 0 |
| | Composé 27$^+$ 15 mg/kg | 20 – 64 | 42,2 | 40 | 0 |
| 1/800 % de leucémies = 100% | TEMOIN VIRUS 1 | 19 – 46 | 24,4 | | |
| | TEMOIN VIRUS 2 | 19 – 35 | 24,4 | | |
| | Composé 27$^{++}$ 15 mg/kg | 48 – 72 | 59,5 | 143,85 | – |

+ = Lot de 10 souris – à cette dose 5 souris/10 sont mortes par toxicité; le calcul d'ILS % a été effectué sur les souris survivantes.

++ = Lot de 10 souris.

Les nouveaux composés peuvent être administrés à l'homme et aux mammifères selon les moyens usuels. Un mode d'administration approprié est la voie injectable, intraveineuse ou intramusculaire. On préfère la voie intraveineuse.

Ainsi l'invention a-t-elle également pour objet des compositions pharmaceutiques convenant à l'administration et contenant au moins un composé de l'invention en association avec un véhicule pharmaceutiquement acceptable. Par exemple, une composition pharmaceutique convenant aux besoins de l'invention est une solution injectable préparée à partir d'un sel pharmaceutiquement acceptable d'un dérivé selon l'invention ou préparée extemporanément à partir d'un dérivé selon l'invention avec une quantité appropriée d'un acide pour obtenir une solution ayant un pH voisin de 7. Une telle solution pourra contenir des additifs usuels dans la formulation gallénique, par exemple des agents tampons.

Au cours des essais pharmacologiques, aucun effet secondaire notable immédiat n'a été trouvé. Les valeurs DL 50 caractéristiques de la toxicité des médicaments ont été déterminées pour un grand nombre de composés représentatifs selon l'invention.

Quant à la posologie envisagée, elle dépendra évidemment du composé particulier utilisé et de la maladie à traiter. Chez l'homme, des doses de l'ordre de 100 mg ou plus composé actif peuvent convenir pour l'adulte.

## TABLEAU XIX

Essai sur le virus de Friend anémiant : relation dose/effet

| | Intervalle de mort | Moyenne du temps de survie | | ILS % | survivantes |
|---|---|---|---|---|---|
| TEMOIN VIRUS 1 | 18 – 31 | 24,4 | 23,6 | – | 0 |
| TEMOIN VIRUS 2 | 18 – 29 | 22,8 | | | |
| Composé 27 5 mg/kg | 25 – 36 | 31,1 | | 31,78 | 0 |
| ,, ,, 10 mg/kg | 32 – 64 | 45,8 | | 103 | 0 |
| ,, ,, 15 mg/kg+ | 33 – 63 | 49,9 | | 122 | 0 |

+ = 2 mortes par toxicité; elles n'entrent pas dans le calcul.

**Revendications**

1. Dérivés des pyrido[4,3-b]carbazoles (ellipticines) substitués en position 1 par une chaîne polyaminée, lesdits dérivés répondant à la formule générale:

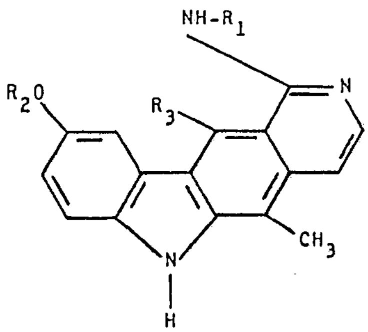

(I)

dans laquelle $R_1$ est un groupement $Y—(CH_2)_n—NR_4R_5$, où Y représente une liaison simple ou le groupe:

$$\begin{array}{c}—CH—\\ |\\ CH_3\end{array}$$

$R_4$ et $R_5$, identiques ou différents, sont l'hydrogène ou un radical alkyle inférieur en $C_1—C_4$, ou encore forment ensemble un cycle pouvant comporter 1 ou 2 atomes d'azote comme hétéroatomes et *n* est un nombre alallant de 1 à 10, notamment de 2 à 7, $R_2$ est un atome d'hydrogène, un groupe alkyle inférieur en $C_1—C_4$ ou un groupe benzyle, et $R_3$ est un atome d'hydrogène ou un groupe $—CH_3$, et les sels pharmaceutiquement acceptables desdits dérivés.

2. Dérivés selon la revendication 1, caractérisés en ce que $R_2$ est le radical $CH_3$, $R_3$ un atome d'hydrogène et $R_1$ l'un des groupes suivants:

$R_1 = (CH_2)_2—NH_2$
$R_1 = (CH_2)_3—NH_2$
$R_1 = (CH_2)_4—NH_2$
$R_1 = (CH_2)_5—NH_2$
$R_1 = (CH_2)_6—NH_2$
$R_1 = (CH_2)_2—N\ (CH_3)_2$
$R_1 = (CH_2)_3—N\ (CH_3)_2$
$R_1 = (CH_2)_3—N\ (C_2H_5)_2$

$$R_1 = \begin{array}{l}—CH—(CH_2)_3—N\ (C_2H_5)_2\\ \ \ |\\ \ CH_3\end{array}$$

$R_1 = —(CH_2)_3—N\ (CH_2\ CH_2)_2—N—(CH_2)_3—NH_2$

3. Dérivés selon la revendication 1, caractérisés en ce que $R_2$ est le radical $CH_2—C_6H_5$, $R_3$ un atome d'hydrogène et $R_1$ l'un des groupes suivants:

$(CH_2)_2—N\ (CH_3)_2$
$(CH_2)_3—NH_2$
$(CH_2)_3—N\ (C_2H_5)_2$

4. Dérivés selon la revendication 1, caractérisés en ce que $R_2$ et $R_3$ sont tous deux le radical $—CH_3$ et $R_1$ l'un des groupes suivants:

$(CH_2)_3—NH_2$
$(CH_2)_3—N\ (CH_3)_2$
$(CH_2)_3—N\ (C_2H_5)_2$

5. Dérivé selon la revendication 1, caractérisé en ce que $R_2$ est le radical $CH_2—C_6H_5$, $R_3$ est le radical $—CH_3$ et $R_1$ est le groupe $(CH_2)3—N\ (C_2H_5)_2$.

6. Dérivé selon la revendication 1, caractérisé en ce que $R_2$ et $R_3$ sont tous deux un atome d'hydrogène et $R_1$ est le groupe $(CH_2)_3—N\ (C_2H_5)_2$.

7. Dérivé selon la revendication 1, caractérisé en ce que $R_2$ est un atome d'hydrogène, $R_3$ est le radical $—CH_3$ et $R_1$ est le groupe $(CH_2)_3—N\ (C_2H_5)_2$.

8. Procédé pour l'obtention des dérivés selon l'une quelconque des revendications 1 à 7, caractérisé par la succession des étapes ci-après:

(1) on fait réagir un chlorure de phényl (substitué en 4) diazonium de formule:

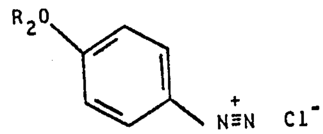

où $R_2$ a la signification indiquée précédemment, sur un cyclohexène à substitution amine en position 1, par exemple un N-morpholino-1-cyclohexène de formule:

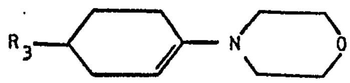

où $R_3$ a la signification indiquée (H ou $-CH_3$).

(2) on transforme l'arylhydrazone obtenue à la première étape, de formule:

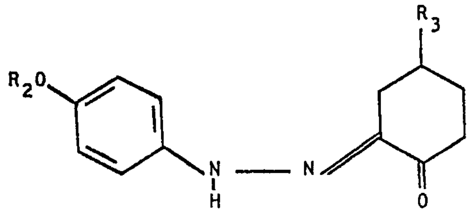

I

où $R_2$ et $R_3$ ont la signification indiquée, par indolisation à chaud et en milieu acide fort.

(3) on acyle l'oxo-1 tétrahydro-1,2,3,4 carbazole, obtenu à la deuxième étape, de formule:

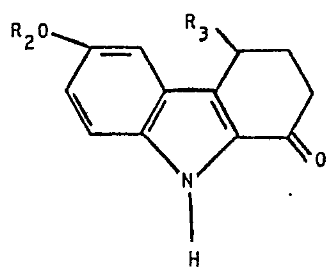

2

où $R_2$ et $R_3$ ont la signification indiquée; à l'aide de formiate d'éthyle en présence d'une base forte.

(4) on éthérifie l'oxo-1 hydroxyméthylène-2 tétrahydro-1,2,3,4 carbazole de formule:

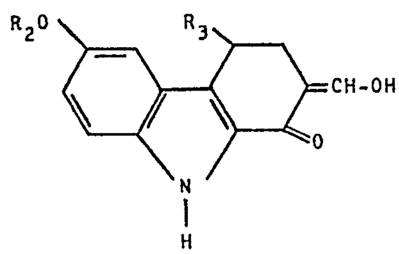

3

où $R_2$ et $R_3$ ont la signification indiquée, à l'aide d'un halogénure d'alkyle, en particulier d'iodure d'isopropyle.

(5) on traite l'oxo-1 alkyloxy-méthylène-2 dihydro-3,4 carbazole, en particulier l'oxo-1 isopropoxyméthylène-2- dihydro-3,4 carbazole, obtenu à la quatrième étape, de formule:

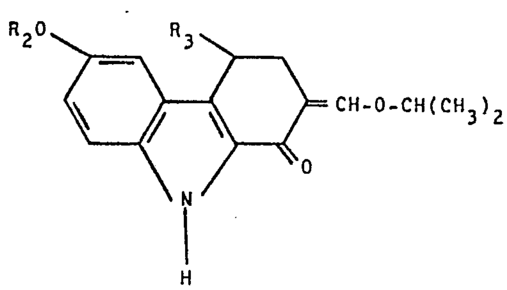

4

où $R_2$ et $R_3$ ont la signification indiquée par quatre équivalents molaires de méthyl lithium, après quoi on opère successivement une hydrolyse acide et une hydrolyse alcaline.

(6) on traite le méthyl-1 formyl-2 dihydro 3,4 carbazole obtenu à la cinquième étape, de formule:

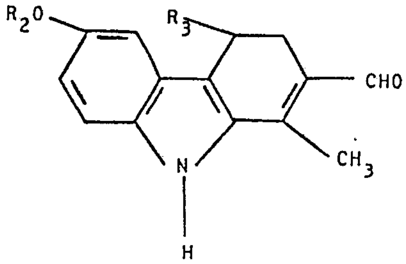

5

où $R_2$ et $R_3$ ont la signification indiquée, par du charbon palladié ou, mieux, par du dioxyde de manganèse, afin d'obtenir l'aromatisation du composé de départ.

(7) on traite l'aldéhyde obtenu à la sixième étape de formule:

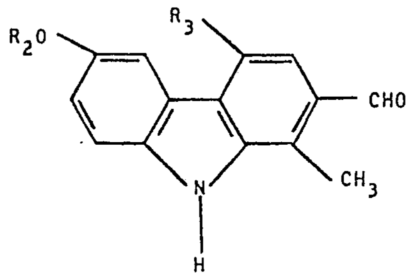

6

par l'acide malonique.

(8) on traite l'acide acrylique obtenu à la septième étape, de formule:

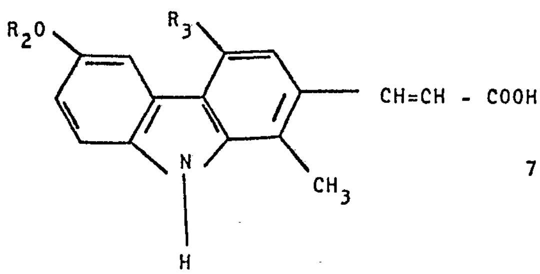

7

par le chloroformiate d'éthyle, puis par l'azoture de sodium.

(9) on traite l'azide obtenu à la huitième étape, de formule:

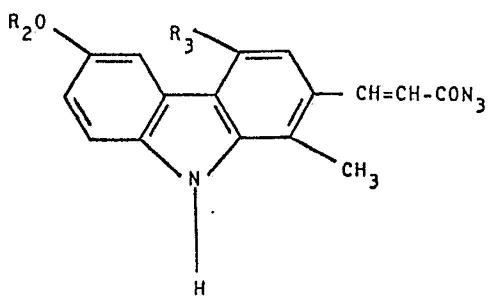

8

par le diphényl éther à chaud.

(10) on traite le dihydro-1,2 oxo-1 méthyl-5 pyrido[4,3-b]carbazole, obtenu à la neuvième étape, de formule

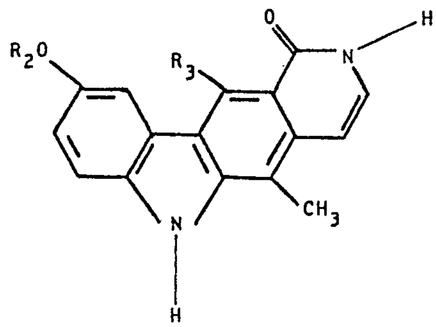

9

par l'oxychlorure de phosphore à chaud.

(11) on traite le chloro-1 méthyl-5 alkyloxy-9 pyrido[4,3-b]carbazole, obtenu à la dixième étape, de formule:

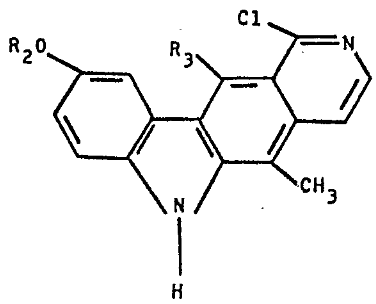

10

par une amine de formule $NH_2$—$R_1$, où $R_1$, $R_2$ et $R_3$ ont la signification indiquée précédemment.

9. Compositions pharmaceutiques, en particulier antitumorales et antileucémiques, contenant, à titre d'agent actif, au moins un des dérivés des pyrido[4,3-b]carbazoles (ellipticines) selon l'une quelconque des revendications 1 à 7, en association avèc un véhicule pharmaceutiquement acceptable et adapté au mode d'administration.

10. Compositions pharmaceutiques selon la revendication 9, conditionnées en vue de l'administration par voie intramusculaire ou intraveineuse.

**Patentansprüche**

1. Derivate von Pyrido[4,3-b]carbazolen (Ellipticinen), die in 1-Stellung durch eine Polyaminkette substituiert sind, wobei die Derivate die allgemeine Formel

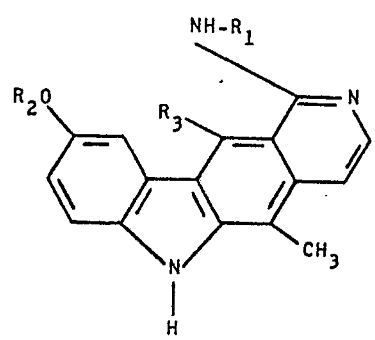

(I)

haben, in der $R_1$ für eine Gruppe der Formel Y—$(CH_2)_n$—$NR_4R_5$ steht, in der Y eine Einfachbindung oder die Gruppe

$$-\underset{\displaystyle CH_3}{\underset{|}{CH}}-$$

ist und $R_4$ und $R_5$, die gleich oder verschieden sind, Wasserstoff oder ein nieder C—$C_4$-Alkylrest sind oder gemeinsam einen Ring bilden, der 1 oder 2 Stickstoffatome als Heteroatome enthalten kann, und *n* eine Zahl von 1 bis 10, insbesondere von 2 bis 7 ist, $R_2$ ein Wasserstoffatom, ein niederer $C_1$—$C_4$-Alkylrest oder ein Benzylrest und $R_3$ ein Wasserstoffatom oder eine —$CH_3$-Gruppe ist, und die pharmazeutisch unbedenklichen Salze dieser Derivate.

2. Derivate nach Anspruch 1, dadurch gekennzeichnet, daß $R_2$ eine $CH_3$-Gruppe, $R_3$ ein Wasserstoffatom und $R_1$ eine der folgenden Gruppen ist:

$R_1 = (CH_2)_2—NH_2$
$R_1 = (CH_2)_3—NH_2$
$R_1 = (CH_2)_4—NH_2$
$R_1 = (CH_2)_5—NH_2$
$R_1 = (CH_2)_6—NH_2$
$R_1 = (CH_2)_2—N\ (CH_3)_2$
$R_1 = (CH_2)_3—N\ (CH_3)_2$
$R_1 = (CH_2)_3—N\ (C_2H_5)_2$

$$R_1 = —\underset{\displaystyle CH_3}{\underset{|}{CH}}—(CH_2)_3—N\,(C_2H_5)_2$$

$R_1 = —(CH_2)_3—N(CH_2CH_2)_2—N—(CH_2)_3—NH_2$

3. Derivate nach Anspruch 1, dadurch gekennzeichnet, daß $R_2$ eine Gruppe der Formel $CH_2—C_6H_5$, $R_3$ ein Wasserstoffatom und $R_1$ eine der folgenden Gruppen ist:

$(CH_2)_2—N\ (CH_3)_2$
$(CH_2)_3—NH_2$
$(CH_2)_3—N(C_2H_5)_2$

4. Derivate nach Anspruch 1, dadurch gekennzeichnet, daß $R_2$ und $R_3$ beide eine $—CH_3$-Gruppe sind und $R_1$ eine der folgenden Gruppen ist:

$(CH_2)_3—NH_2$
$(CH_2)_3—N\ (CH_3)_2$
$(CH_2)_3—N\ (C_2H_5)_2$

5. Derivate nach Anspruch 1, dadurch gekennzeichnet, daß $R_2$ eine Gruppe der Formel $CH_2—C_6H_5$, $R_3$ eine $—CH_3$-Gruppe und $R_1$ eine Gruppe der Formel $(CH_2)_3—N(C_2H_5)_2$ ist.

6. Derivate nach Anspruch 1, dadurch gekennzeichnet, daß $R_2$ und $R_3$ beide ein Wasserstoffatom sind und $R_1$ eine Gruppe der Formel $(CH_2)_3—N(C_2H_5)_2$ ist.

7. Derivate nach Anspruch 1, dadurch gekennzeichnet, daß $R_2$ ein Wasserstoffatom, $R_3$ eine $—CH_3$-Gruppe und $R_1$ eine Gruppe der Formel $(CH_2)_3—N(C_2H_5)_2$ ist.

8. Verfahren zur Herstellung von Derivaten nach Anspruch 1 bis 7, gekennzeichnet durch die Aufeinanderfolge der folgenden Stufen:

2) man setzt ein (in 4-Stellung substituiertes) Phenyldiazoniumchlorid der Formel

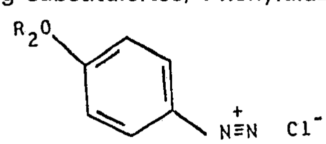

in der $R_2$ die genannte Bedeutung hat, mit einem Cyclohexen mit Aminsubstitution in 1-Stellung beispielsweise einem N-Morpholin-1-cyclohexen der Formel

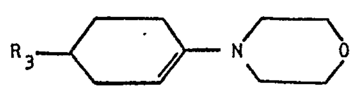

in der $R_3$ die genannte Bedeutung hat (H oder $—CH_3$), um;

2) man wandelt das in der ersten Stufe erhaltene Arylhydrazon der Formel

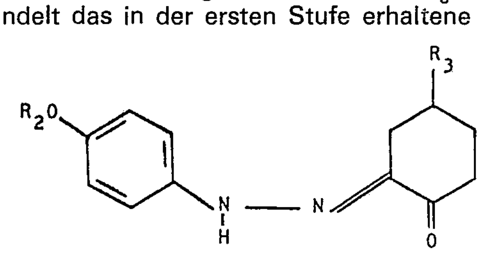

I

in der $R_2$ und $R_3$ die genannten Bedeutungen haben, durch Indolisierung in der Wärme in einem stark sauren Medium um;

3) man acyliert das in der zweiten Stufe erhaltene 1-Oxo-1,2,3,4-carbazol der Formel

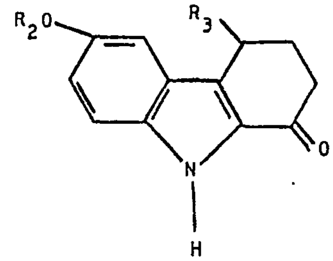

2

in der $R_2$ und $R_3$ die genannten Bedeutungen haben, mit Äthylformiat in Gegenwart einer starken Base;

4) man veräthert 1-Oxo-2-hydroxymethylen-1,2,3,4-tetrahydrocarbazol der Formel

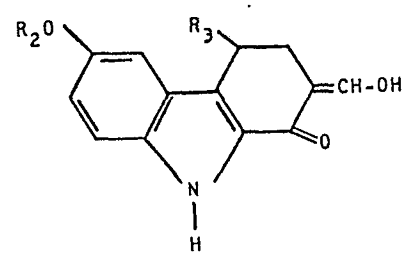

3

in der $R_2$ und $R_3$ die genannten Bedeutungen haben, mit einem Alkylhalogenid, insbesondere Isopropyljodid;

5) man behandelt das in der vierten Stufe erhaltene 1-Oxo-2-alkyloxymethylen-3,4-dihydrocarbazol, insbesondere 1-Oxo-2-isopropoxymethylen-3,4-dihydrocarbazol der Formel

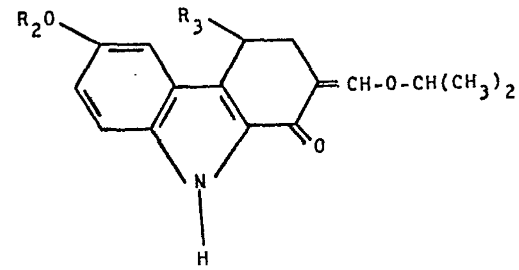

4

in der $R_2$ und $R_3$ die genannten Bedeutungen haben, mit vier molaren Äquivalenten von Lithiummethyl, worauf man nacheinander eine saure Hydrolyse und eine alkalische Hydrolyse vornimmt;

6) man behandelt das in der fünften Stufe erhaltene 1-Methyl-2-formyl-3,4-dihydrocarbazol der Formel

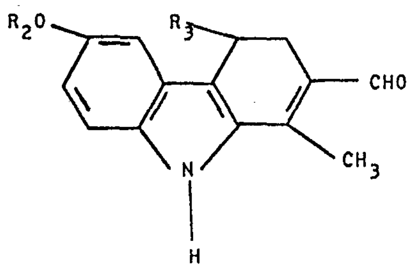

5

in der $R_2$ und $R_3$ die genannten Bedeutungen haben, mit Palladiumkohle oder besser mit Mangandioxid, um Aromatisierung der Ausgangsverbindung zu erzielen;

7) man behandelt den in der sechsten Stufe erhaltenen Aldehyd der Formel

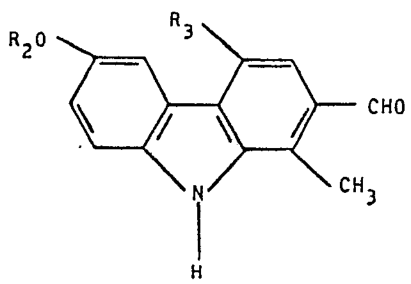

6

mit Malonsäure;

8) man behandelt die in der siebten Stufe erhaltene Acrylsäure der Formel

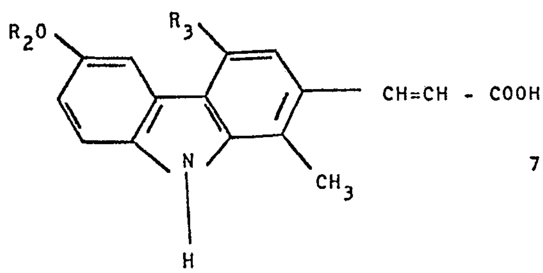

7

mit Äthylchlorformiat und dann mit Natriumnitrid;

9) man behandelt das in der achten Stufe erhaltene Azid der Formel

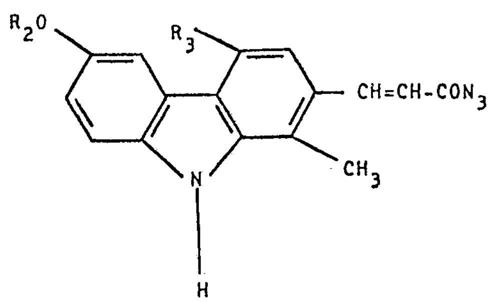

8

heiß mit Diphenyläther;

10) man behandelt das in der neunten Stufe erhaltene 1,2-Dihydro-1-oxo-5-methylpyrido[4,3-b]-carbazol der Formel

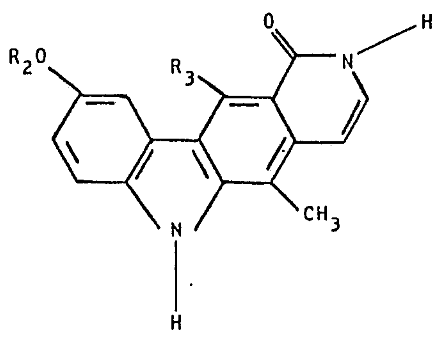

9

heiß mit Phosphoroxychlorid;

11) man behandelt das in der zehnten Stufe erhaltene 1-Chlor-5-methyl-9-alkyloxypyrido[4,3-b]-carbazol der Formel

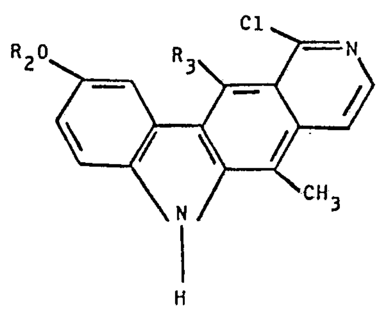

10

mit einem Amin der Formel $NH_2—R_1$, wobei $R_1$, $R_2$ und $R_3$ die vorstehend genannten Bedeutungen haben.

9. Arzneimittelzubereitungen, insbesondere Antitumormittel und antileukämische Mittel, enthaltend als Wirkstoff wenigstens eines der Derivate der Pyrido[4,3-b]carbazole (Ellipticine) nach irgendeinem der Ansprüche 1 bis 7 in Verbindung mit einem pharmazeutisch unbedenklichen Träger, der dem Darreichungsweg angepasst ist.

10. Arzneimittelzubereitungen nach Anspruch 9, die im Hinblick auf die intramuskuläre oder intravenöse Verabreichung konditioniert sind.

## Claims

1. Derivatives of pyrido[4,3-b]carbazoles (ellipticines) substituted at the 1 position by a polyamine chain, said derivatives having the general formula:

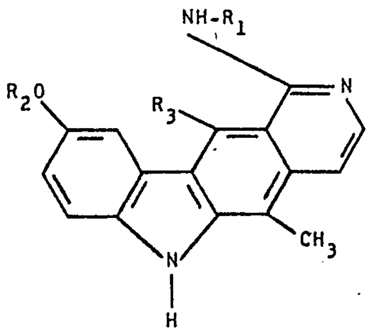

(I)

in which $R_1$ is a $Y—(CH_2)_n—NR_4R_5$ group, where Y represents a single bond or the group

$$—CH(CH_3)—$$

$R_4$ and $R_5$, identical or different, are hydrogen or $C_1—C_4$ lower alkyl radical, or again form together a ring which can include one or two nitrogen atoms as heteroatoms, and *n* is a number ranging from 1 to 10 notably from 2 to 7, $R_2$ is a hydrogen atom, a $C_1—C_4$ lower alkyl group or a benzyl group, $R_3$ is a hydrogen atom or a $—CH_3$ group, and the pharmaceutically acceptable salts of said derivatives.

2. Derivatives according to claim 1, wherein $R_2$ is the radical $CH_3$, $R_3$ is a hydrogen atom and $R_1$ one of the following groups:

$R_1 = (CH_2)_2—NH_2$
$R_1 = (CH_2)_3—NH_2$
$R_1 = (CH_2)_4—NH_2$
$R_1 = (CH_2)_5—NH_2$
$R_1 = (CH_2)_6—NH_2$
$R_1 = (CH_2)_2—N(CH_3)_2$
$R_1 = (CH_2)_3—N(C_2H_5)_2$

$R_1 = —CH(CH_3)—(CH_2)_3—N(C_2H_5)_2$

$R_1 = —(CH_2)_3—N(CH_2—CH_2)_2—N—(CH_2)_3—NH_2$.

3. Derivatives according to claim 1, wherein $R_2$ is the $CH_2—C_6H_5$ radical, $R_3$ is a hydrogen atom and $R_1$ one of the following groups:

$(CH_2)_2—N(CH_3)_2$
$(CH_2)_3—NH_2$
$(CH_2)_3—N(C_2H_5)_2$.

4. Derivatives according to claim 1, wherein $R_2$ and $R_3$ are both the $—CH_3$ radical and $R_1$ one of the following groups:

$(CH_2)_3—NH_2$
$(CH_2)_3—N\ (CH_3)_2$
$(CH_2)_3—N\ (C_2H_5)_2$.

5. Derivatives according to claim 1, wherein $R_2$ is the $CH_2—C_6H_5$ radical, $R_3$ is the $—CH_3$ radical and $R_1$ is the group $(CH_2)_3—N\ (C_2H_5)_2$.

6. Derivative according to claim 1, wherein $R_2$ and $R_3$ are both a hydrogen atom and $R_1$ is the group $(CH_2)_3—N\ (C_2H_5)_2$.

7. Derivative according to claim 1, wherein $R_2$ is a hydrogen atom, $R_3$ is the radical $—CH_3$ and $R_1$ is the group $(CH_2)_3—N\ (C_2H_5)_2$.

8. Process for preparing derivatives according to claim 1, characterized in that it comprises the following succession of steps:

(1) a phenyl diazonium chloride (substituted at the 4 position) of the formula:

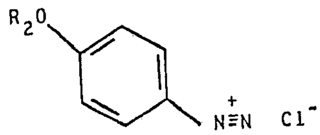

where $R_2$ has the previously indicated meaning, is reacted with a cyclohexene with an amine substitution at the 1 position; for example an N-morpholino-1-cyclohexene of the formula:

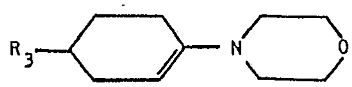

(2) the arylhydrazone obtained in the first step, of the formula:

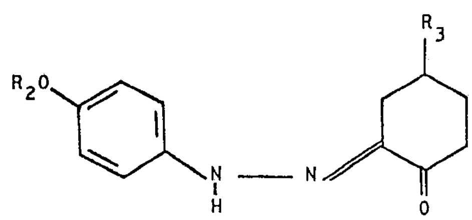

I

where $R_2$ and $R_3$ have the indicated meaning, is converted by indolisation in the hot and in a strongly acid medium;

(3) the 1-oxo-1,2,3,4-tetrahydro-carbazole, obtained in the second step of the formula:

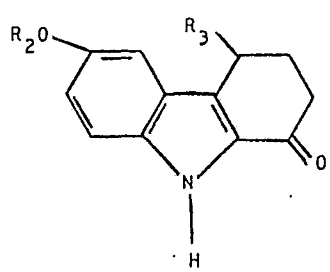

2

where $R_2$ and $R_3$ have the indicated meaning, is acylated by means of ethyl formate in the presence of a strong base;

(4) the 1-oxo-2-hydroxymethylene-1,2,3,4-tetrahydro carbazole of the formula:

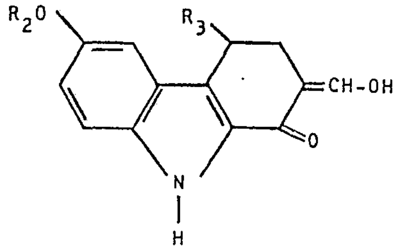

3

where $R_2$ and $R_3$ have the indicated meaning, is etherified by means of an alkyl halide; particularly of isopropyl iodide;

(5) the 1-oxo-2-alkyloxy-methylene-3,4-dihydro-carbazole, in particular 1-oxo-2-isopropoxy-methylene-3,4-dihydro-carbazole, obtained in the fourth step, of the formula:

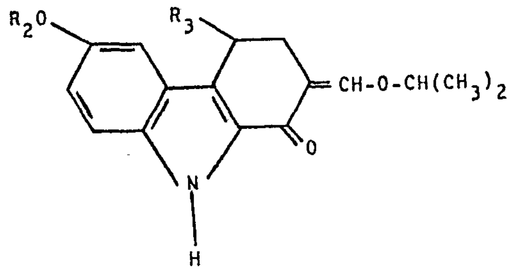

4

where $R_2$ and $R_3$ have the indicated meaning is treated with four molar equivalents of methyl lithium, after which an acid hydrolysis and an alkaline hydrolysis are successively carried out;

(6) the 1-methyl-2-formyl-3,4-dihydro-carbazole obtained in the fifth step, of the formula:

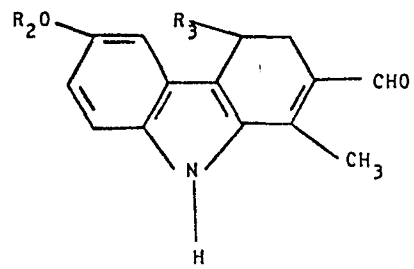

5

where $R_2$ and $R_3$ have the indicated meaning, is treated with palladium charcoal or, better with manganese dioxide, in order to obtain the aromatization of the starting compound;

(7) the aldehyde obtained in the sixth step, of the formula:

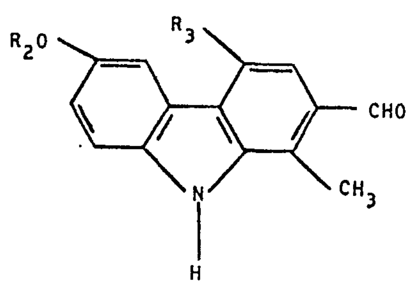

6

is treated with malonic acid;

(8) the acrylic acid obtained in the seventh step, of the formula:

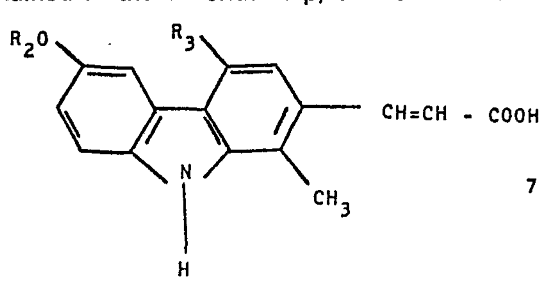

7

is treated with ethyl chloroformate, and then with sodium azide;

(9) the azide obtained in the eighth step of the formula:

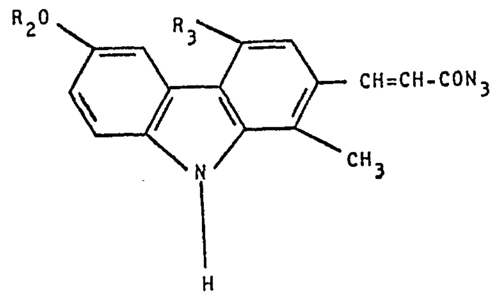

8

is treated with diphenyl ether in the hot;

(10) the 1,2-dihydro-1-oxo-5-methyl-pyrido[4,3-b]carbazole, obtained in the ninth step, of the formula:

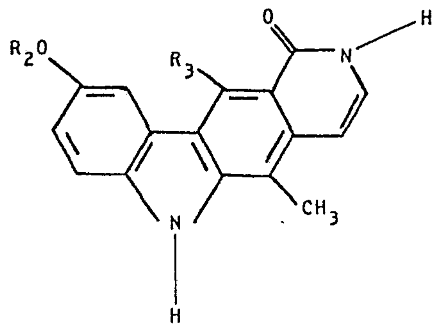

9

is treated with phosphorus oxychloride in the hot;

(11) the 1-chloro-5-methyl-9-alkyloxy-pyrido[4,3-b]carbazole, obtained in the tenth step, of the formula:

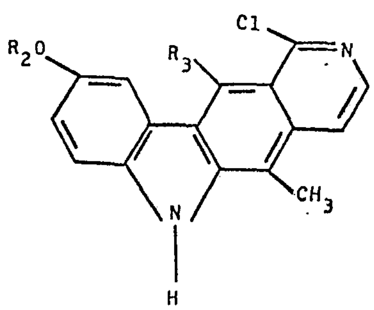

10

is treated with an amine of the formula $NH_2$—$R_1$, where $R_1$, $R_2$ and $R_3$ have the previously indicated meaning.

9. Pharmaceutical compositions, in particular antitumoral and antileukemia compositions, containing as active agent, at least one of the derivatives of pyrido[4,3-b]carbazoles (ellipticines) according to any one of claims 1 to 7, in association with a pharmaceutically acceptable vehicle and adapted for the method of administration.

10. Pharmaceutical compositions according to claim 9, conditioned for administration by the intramuscular or intravenous route.

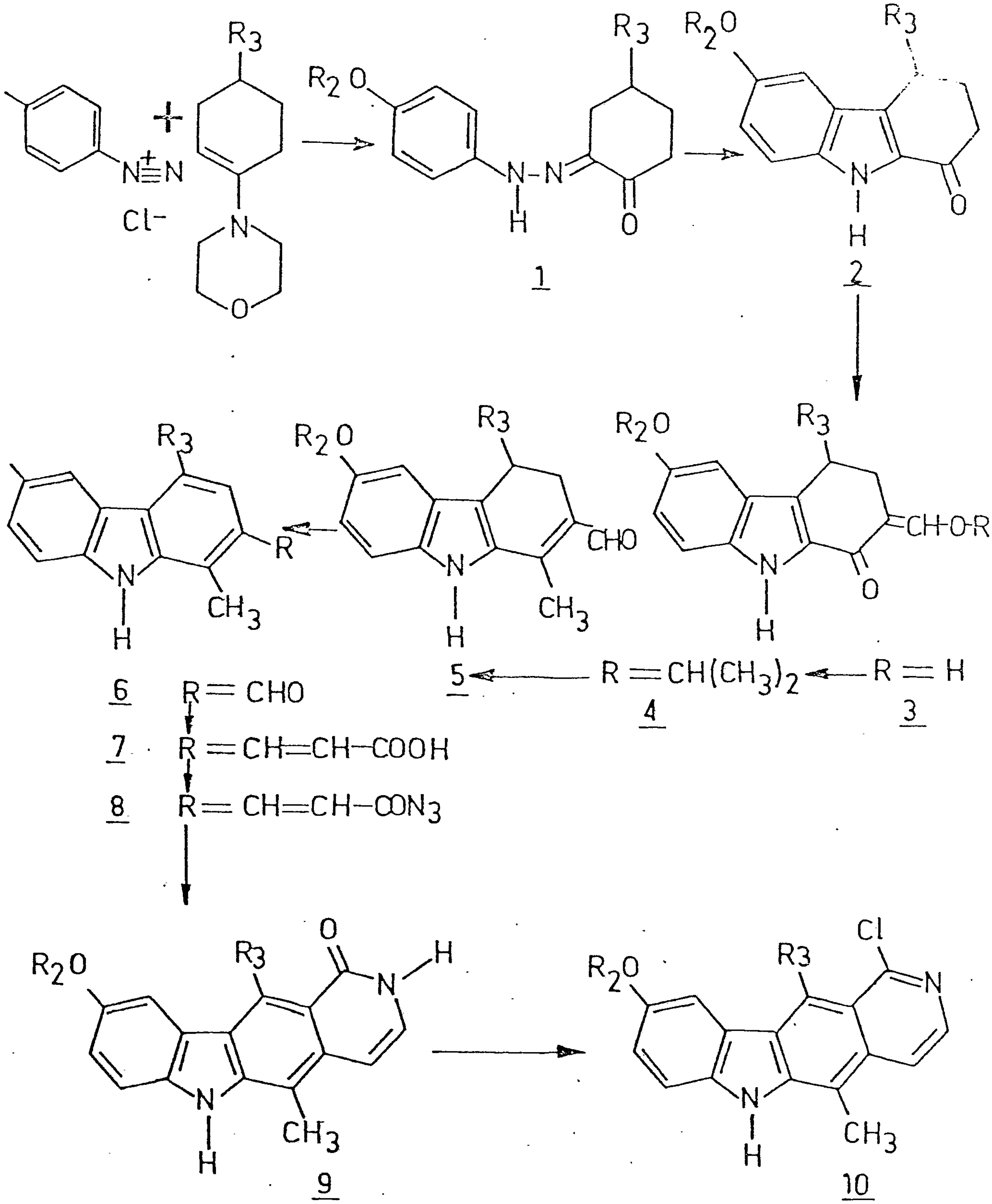

1

2

5 ← R = $CH(CH_3)_2$ ← R = H

4 3

6 R = CHO

7 R = CH=CH–COOH

8 R = CH=CH–$CON_3$

9

10